(19) Europäisches Patentamt
European Patent Office
Office européen des brevets

(11) EP 3 048 454 A1

(12) EUROPEAN PATENT APPLICATION

(43) Date of publication:
27.07.2016 Bulletin 2016/30

(51) Int Cl.:
G01S 7/52 (2006.01)     G01S 15/10 (2006.01)

(21) Application number: 15182839.9

(22) Date of filing: 28.08.2015

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(30) Priority: 21.01.2015 KR 20150009876

(71) Applicant: Samsung Electronics Co., Ltd
Gyeonggi-do 443-742 (KR)

(72) Inventors:
• KIM, Bae Hyung
  Gyeonggi-do (KR)
• KIM, Kyu Hong
  Seoul (KR)
• PARK, Su Hyun
  Gyeonggi-do (KR)
• SONG, Jong Keun
  Gyeonggi-do (KR)
• LEE, Seung Heun
  Gyeonggi-do (KR)

(74) Representative: Grünecker Patent- und Rechtsanwälte
PartG mbB
Leopoldstraße 4
80802 München (DE)

(54) **ULTRASOUND PROBE, ULTRASOUND DIAGNOSTIC APPARATUS HAVING THE SAME AND METHOD OF GENERATING ULTRASOUND SIGNAL**

(57)     An ultrasound probe includes a transmission unit configured to generate a transmission signal; and a modulator configured to generate a modulation signal having a pulse width proportional to a size of an amplitude of the transmission signal at a peak level and a base level for each period of the transmission signal.

**FIG. 8**

**Description**

BACKGROUND

**1. Field**

Apparatuses and methods consistent with exemplary embodiments relate to an ultrasound probe and an ultrasound diagnostic apparatus.

**2. Description of the Related Art**

An ultrasound diagnostic apparatus emits an ultrasound signal to a target area inside a body from a surface of an object and obtains a tomographic image of a soft tissue or an image of blood flow using information on a reflected ultrasound signal (ultrasound echo signal) in a noninvasive manner.

The ultrasound diagnostic apparatus is advantageous in that it is small and inexpensive, can display an image in real time, and has a higher safety level, compared to other image diagnostic apparatuses such as an X-ray imaging apparatus, a magnetic resonance imaging apparatus, and a nuclear medicine diagnostic apparatus. Due to such advantages, the ultrasound diagnostic apparatus is being widely used for medical diagnostics.

SUMMARY

One or more exemplary embodiments provide an ultrasound probe configured to increase matching of a transmission signal with respect to a frequency spectrum of a transducer element and modulate a transmission signal to increase transmission power efficiency and a signal-to-noise ratio, and an ultrasound diagnostic apparatus having the same and a method of generating an ultrasound signal.

According to an aspect of an exemplary embodiment, there is provided an ultrasound probe, including: a transmission unit configured to generate a transmission signal; and a modulator configured to generate a modulation signal having a pulse width proportional to a size of an amplitude of the transmission signal at a peak level and a base level for each period of the transmission signal.

The transmission unit may output a transmission signal in which a first transmission signal and a second transmission signal that is shifted by a predetermined time from the first transmission signal are synthesized.

The transmission unit may include a first transmission element configured to output a first transmission signal; and a second transmission element configured to output a second transmission signal that is shifted by a predetermined time from the first transmission signal.

The second transmission signal may be shifted by a time corresponding to 1/4 of a period of the first transmission signal from the first transmission signal.

The modulator may generate a modulation signal having a pulse width proportional to a size of an amplitude of a synthesized signal at a peak level and a base level for each period of the signal in which the first transmission signal and the second transmission signal are synthesized.

The modulator may include a first modulator configured to generate a first modulation signal having a pulse width proportional to a size of an amplitude of the first transmission signal at a peak level and a base level for each period of the first transmission signal; and a second modulator configured to generate a second modulation signal having a pulse width proportional to a size of an amplitude of the second transmission signal at a peak level and a base level for each period of the second transmission signal.

The ultrasound probe may further include an adder configured to synthesize the first modulation signal and the second modulation signal.

According to another aspect of an exemplary embodiment, there is provided an ultrasound probe, including: at least one modulator configured to generate a modulation signal having a pulse width proportional to a size of an amplitude of a transmission signal at a peak level and a base level for each period of the transmission signal; and a pulser configured to generate a transmitting pulse corresponding to the modulation signal and apply the pulse to a transducer array.

According to still another aspect of an exemplary embodiment, there is provided a method of generating an ultrasound signal, including: generating a transmission signal; and generating a modulation signal having a pulse width proportional to a size of an amplitude of a transmission signal at a peak level and a base level for each period of the transmission signal.

According to yet another aspect of an exemplary embodiment, there is provided a method of generating an ultrasound signal, including: generating a modulation signal having a pulse width proportional to a size of an amplitude of a transmission signal at a peak level and a base level for each period of the transmission signal; and generating a transmitting pulse corresponding to the modulation signal and applying the pulse to a transducer array.

According to yet another aspect of an exemplary embodiment, there is provided an ultrasound diagnostic apparatus, including: an ultrasound probe configured to generate a transmitting pulse having a pulse width proportional to a size of an amplitude of an transmission signal at a peak level and a base level for each period of the transmission signal; and a workstation configured to generate and display an ultrasound image when the ultrasound probe receives an ultrasound echo signal.

BRIEF DESCRIPTION OF THE DRAWINGS

The above and/or other aspects will become more apparent by describing certain exemplary embodiments with reference to the accompanying drawings, in which:

FIG. 1 is a diagram illustrating an ultrasound diagnostic apparatus according to an exemplary embodiment;
FIG. 2 is a control block diagram illustrating an ultrasound diagnostic apparatus according to an exemplary embodiment;
FIG. 3 is a control block diagram illustrating a configuration of a main body of an ultrasound diagnostic apparatus according to an exemplary embodiment;
FIG. 4 is a diagram illustrating a configuration of a transmitter according to an exemplary embodiment;
FIG. 5 is a diagram illustrating an operation of transmit beamforming;
FIG. 6 is a diagram illustrating a delay of a transmission signal applied when transmit beamforming is performed;
FIG. 7 is a diagram illustrating a process of generating a waveform in a transmission unit of an ultrasound probe according to an exemplary embodiment;
FIG. 8 is a diagram illustrating a process of modulating a transmission signal in a transmitter according to an exemplary embodiment;
FIGS. 9A and 9B are diagrams illustrating waveforms of a transmission signal and a modulation signal generated in a transmitter according to an exemplary embodiment;
FIG. 10 is a diagram illustrating a waveform of a signal in which a signal that is modulated in a transmitter according to an exemplary embodiment and a signal that is shifted by a predetermined time from the modulation signal are synthesized;
FIG. 11 is a diagram illustrating a cosine waveform for calculating a shift time of a modulation signal in a transmitter according to an exemplary embodiment;
FIG. 12 is a diagram illustrating a configuration of a transmitter according to an exemplary embodiment;
FIG. 13 is a diagram illustrating modulation of a transmission signal in a transmitter according to an exemplary embodiment;
FIG. 14 is a diagram illustrating a configuration of a transmitter according to an exemplary embodiment;
FIG. 15 is a diagram illustrating modulation of a transmission signal in a transmitter according to an exemplary embodiment;
FIG. 16 is a diagram illustrating a configuration of a receiver according to an exemplary embodiment;
FIG. 17 is a diagram illustrating receive beamforming in a receiver according to an exemplary embodiment;
FIG. 18 is a diagram illustrating a delay of a reception signal applied when receive beamforming is performed;
FIG. 19 is a block diagram illustrating a configuration of an ultrasound diagnostic apparatus according to an exemplary embodiment;
FIGS. 20 and 21 are diagrams illustrating a transmitter and a receiver of an ultrasound probe according to an exemplary embodiment;
FIG. 22 is a flowchart illustrating a method of generating an ultrasound signal according to an exemplary embodiment;
FIG. 23 is a flowchart illustrating a method of generating an ultrasound signal according to an exemplary embodiment; and
FIG. 24 is a flowchart illustrating a method of generating an ultrasound signal according to an exemplary embodiment.

DETAILED DESCRIPTION

Certain exemplary embodiments are described in greater detail below with reference to the accompanying drawings.

In the following description, the same drawing reference numerals are used for the same elements even in different drawings. Thus, description of the same elements is not repeated. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

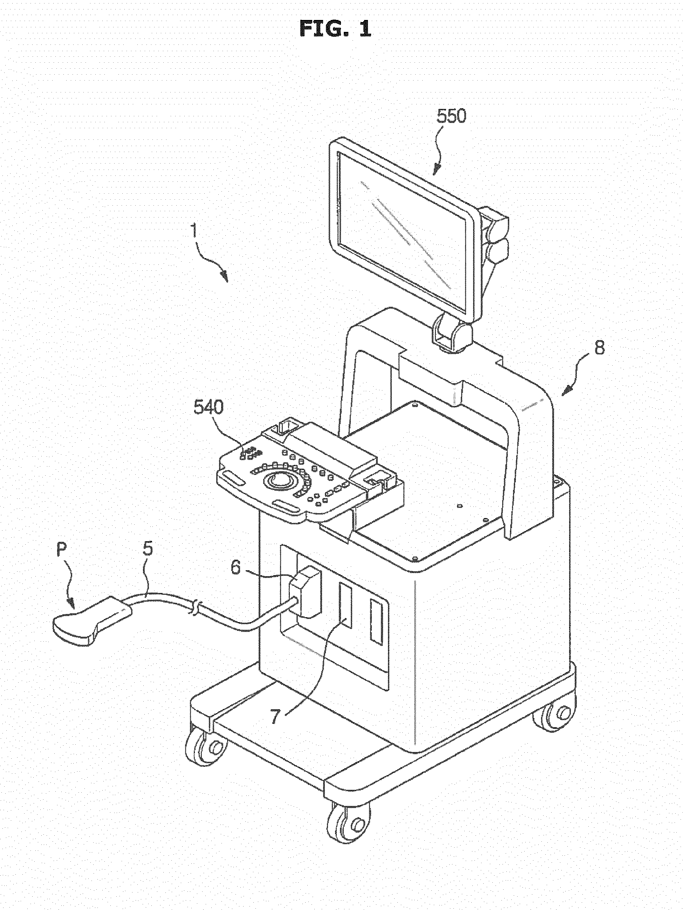

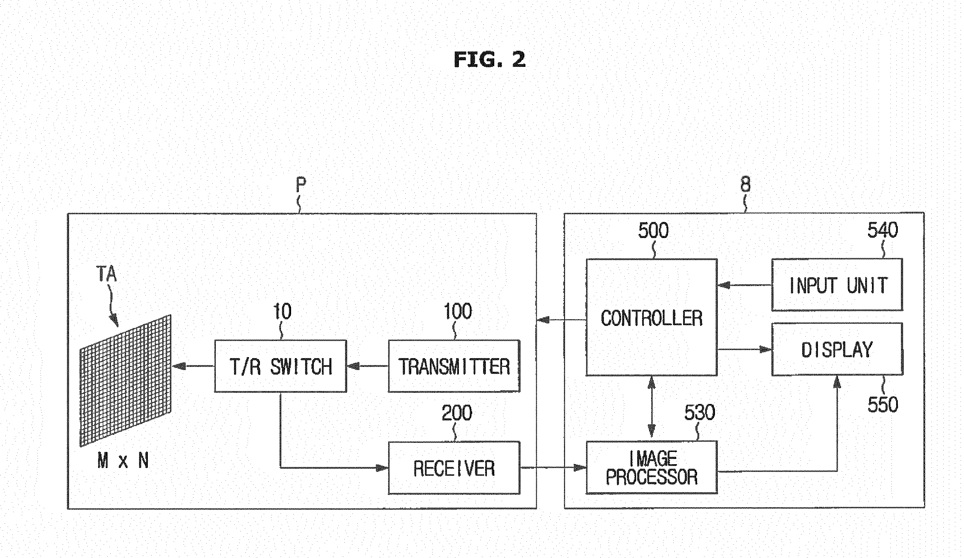

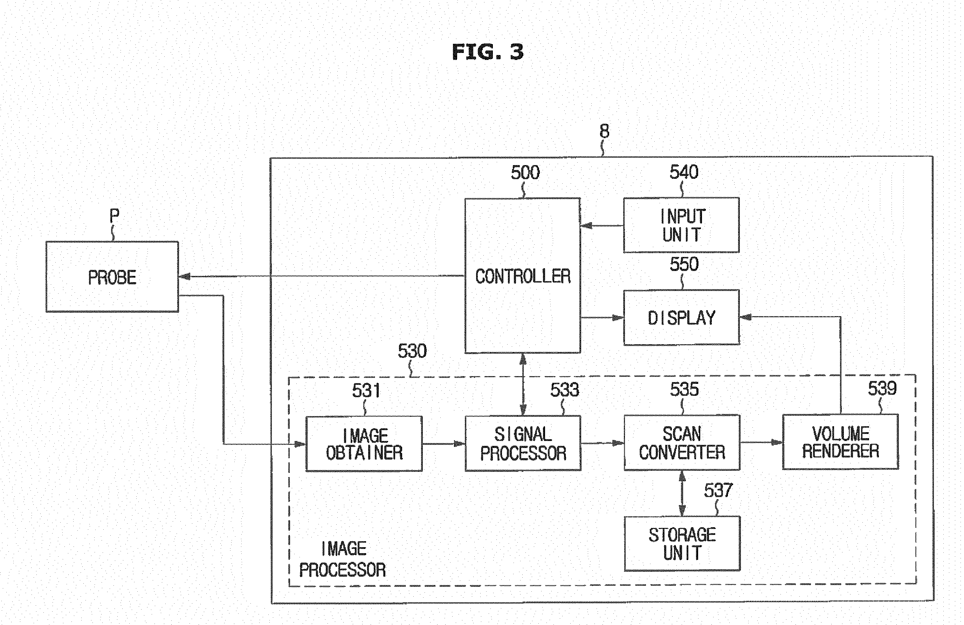

FIG. 1 is a diagram illustrating an ultrasound diagnostic apparatus according to an exemplary embodiment. FIG. 2 is a control block diagram illustrating an ultrasound diagnostic apparatus according to an exemplary embodiment. FIG. 3 is a control block diagram specifically illustrating a configuration of a main body of an ultrasound diagnostic apparatus according to an exemplary embodiment.

As illustrated in FIG. 1, an ultrasound diagnostic apparatus 1 includes an ultrasound probe P configured to transmit an ultrasound to an object, receive an echo ultrasound from the object, and convert the echo ultrasound into an electrical signal, and a main body 8 that is connected to the ultrasound probe, includes an input unit 540 and a display 550, and displays an ultrasound image.

The ultrasound probe is connected to the main body of the ultrasound diagnostic apparatus through a cable 5 which receives various signals for controlling the ultrasound probe or may deliver an analog signal or a digital signal corresponding to the ultrasound echo signal received by the ultrasound probe to the main body. However, an exemplary embodiment of the ultrasound probe is not limited thereto, but a wireless probe may be implemented to transmit and receive a signal via a network between the ultrasound probe and the main body. One end of the cable is connected to the ultrasound probe, and a connector 6 detachably connectable to a slot 7 of the main body may be provided at the other end of the cable. The main body and the ultrasound probe may transmit and receive a control command or data using the cable. For example, when a user inputs information on a focal depth, a size or a shape of an aperture, a steering angle or the like through the input unit, these pieces of information may be delivered to the ultrasound probe through the cable and used for performing the beamforming of a transmitter 100 and a receiver 200. Also, when the user inputs information on an imaging mode such as coded excitation imaging or harmonic imaging through the input unit, these pieces of information are delivered to the ultrasound probe through the cable, and the ultrasound probe may modulate a transmission signal through pulse width modulation and generate a transmitting pulse. When the ultrasound probe is implemented as the wireless probe, the ultrasound probe is connected to the main body via a wireless network with no cable. When the ultrasound probe is connected to the main body via the wireless network, the main body and the ultrasound probe may transmit and receive the above-described control command or data.

As illustrated in FIG. 2, the main body may include a controller 500, an image processor 530, an input unit and a display 550. The controller controls overall operations of the ultrasound diagnostic apparatus. Specifically, the controller generates a control signal for controlling components of the ultrasound diagnostic apparatus, for example, the transmitter 100, a transmit/receive (T/R) switch 10, a receiver 200, the image processor, the display and the like illustrated in FIG. 2, and controls operations of the above-described components. In particular, the controller calculates a delay profile of a plurality of ultrasound transducer elements of a 2D ultrasound transducer array TA, for example, including MxN transducer elements, and calculates a time delay value resulting from a distance difference between the plurality of ultrasound transducer elements included in the 2D ultrasound transducer array and a focal point of the object based on the calculated delay profile. The controller accordingly controls a beamformer to generate a signal. The controller generates a control command for each of the components of the ultrasound diagnostic apparatus according to the user's instruction or command input through the input unit and controls the ultrasound diagnostic apparatus.

The image processor generates a 3D ultrasound image of a target area inside the object based on an ultrasound signal focused through the receiver.

As illustrated in FIG. 3, the image processor may include an image obtainer 531, a signal processor 533, a scan converter 535, a storage unit 537 and a volume renderer 539. The image obtainer generates a coherent 2D image or 3D image of the target area inside the object based on the ultrasound signal focused through the receiver. The signal processor converts coherent image information formed by the image obtainer into ultrasound image information according to a diagnostic mode such as a brightness mode (B-mode) or a Doppler mode (D-mode). For example, when the diagnostic mode is set as the B-mode, the signal processor performs a process such as an analog to digital (A/D) converting process on coherent image information, and generates ultrasound image information for a B-mode image in real time. Also, when the imaging mode is set as a D-mode, the signal processor extracts phase change information from the ultrasound signal, calculates information on a blood flow corresponding to each point of a cross section to be imaged such as a speed, power, and a distribution, and generates ultrasound image information for a D-mode image in real time. The scan converter converts the converted ultrasound image information input from the signal processor or the converted ultrasound image information stored in the storage unit into a general video signal for the display, and transmits the result to the volume renderer. The storage unit stores the ultrasound image information that was converted through the signal processor temporarily or non-temporarily. The volume renderer performs volume rendering based on the video signal transmitted from the scan converter, corrects the rendered image information, generates the final result image, and then transmits the generated result image to the display.

The input unit may be provided for the user to input a command for an operation of the ultrasound diagnostic apparatus. The user may input or set a diagnostic mode selecting command such as an ultrasound diagnosis start command, an amplitude mode (A-mode), a B-mode, a color mode, a D-mode, a motion mode (M-mode), etc., and region of interest (ROI) setting information including a size and a position of the ROI through the input unit. The input unit may include various devices such as a keyboard, a mouse, a trackball, a tablet or a touch screen module that may be used for the user to input data, an instruction or a command. The display displays menus or instructions for ultrasound procedure, an ultrasound image obtained by an ultrasound diagnostic process and the like. The display displays an ultrasound image of the target area inside the object generated by the image processor. The ultrasound image displayed on the display may be an ultrasound image in the A-mode, an ultrasound image in the B-mode, or a 3D stereoscopic ultrasound image. The display may be implemented as various known display components such as a cathode ray tube (CRT) or a liquid crystal display (LCD).

The ultrasound transducer array is provided at an end of the ultrasound probe. The ultrasound transducer array refers to a plurality of ultrasound transducer elements that are disposed in the form of an array. The ultrasound transducer array according to an exemplary embodiment has the form of a 2D array. The ultrasound transducer array vibrates due to a pulse signal or an alternating current applied to the ultrasound transducer array and generates an ultrasound. The generated ultrasound is transmitted to the target area inside the object. In this case, the ultrasound generated in the ultrasound transducer array may be transmitted to be focused on a plurality of target areas inside the object. In other words, the generated ultrasound may be multi-focused on the plurality of target areas and then transmitted. The ultrasound generated in the ultrasound transducer array is reflected from the target area inside the object and is returned to the ultrasound transducer array again. The ultrasound transducer array receives an echo ultrasound that is reflected from at least one target area and returned. When the echo ultrasound arrives at the ultrasound transducer array, the ultrasound transducer array vibrates at a predetermined frequency corresponding to a frequency of the echo ultrasound, and outputs an alternating current of a frequency corresponding to a vibration frequency of the ultrasound transducer array. Therefore, the ultrasound transducer array may convert the received echo ultrasound into a predetermined electrical signal.

Since each of the elements receives the echo ultrasound and outputs an electrical signal, the ultrasound transducer array may output electrical signals of a plurality of channels. The number of channels may be the same as the number of ultrasound transducer elements of the ultrasound transducer array. The ultrasound transducer element may include a piezoelectric vibrator or a thin film. When an alternating current is applied from a power source, the piezoelectric vibrator or the thin film vibrates at a predetermined frequency according to the applied alternating current, and generates an ultrasound of a predetermined frequency according to the vibration frequency. On the other hand, when the echo ultrasound of a predetermined frequency arrives at the piezoelectric vibrator or the thin film, the piezoelectric vibrator or the thin film vibrates according to the echo ultrasound, and outputs an alternating current of a frequency corresponding to the vibration frequency. The ultrasound transducer may be implemented as any of a magnetostrictive ultrasonic transducer using a magnetostrictive effect of a magnetic substance, a piezoelectric ultrasonic transducer using a piezoelectric effect of a piezoelectric material, and a capacitive micromachined ultrasonic transducer (cMUT) that transmits and receives an ultrasound using vibrations of several hundreds or thousands of micromachined thin films. Also, other types of transducers capable of generating an ultrasound according to an electrical signal or an electrical signal according to the ultrasound may be exemplary ultrasound transducers.

The T/R switch determines a transceiving state of the ultrasound probe. When the T/R switch sets a transmitting state, the transmitter applies a transmitting pulse to the transducer array, and enables the transducer array to transmit the ultrasound signal to the target area inside the object.

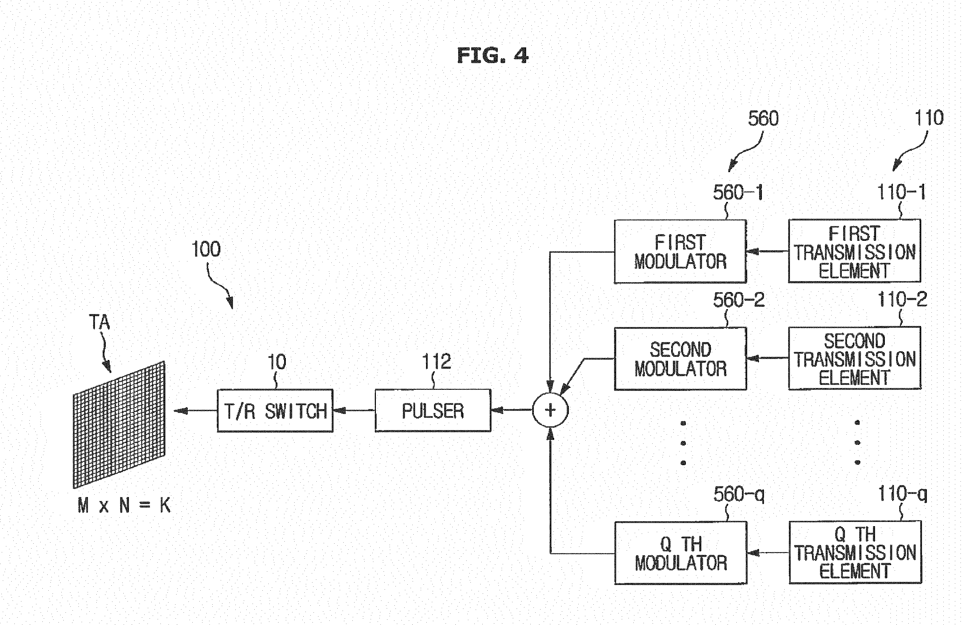

FIG. 4 is a control block diagram illustrating a detailed configuration of the transmitter according to an exemplary embodiment. As illustrated in FIG. 4, the transmitter includes a transmission unit 110, a modulator 560, and a pulser 112. The transmission unit 110 may include a first transmission element 110-1 and a second through Qth transmission elements 110-2 through 110-q. The modulator 560 may include a first modulator 560-1 and a second through Qth modulators 560-2 through 560-q.

The transmitter according to an exemplary embodiment modulates a transmission signal through a modified pulse width modulation method in order to provide an imaging method such as coded excitation imaging and harmonic imaging. The coded excitation imaging is an imaging method in which a degree of penetration and a resolution of the ultrasound are increased, and a clear image of a target area in a deep region of the object may be obtained. In order to increase a degree of penetration of the ultrasound, an output of the ultrasound should be increased. In the coded excitation imaging, in order to increase an output of the ultrasound, a burst pulse formed of a plurality of pulses rather than a single pulse is output. When the burst pulse is output, a degree of penetration of the ultrasound may increase, but a resolution in an axial direction decreases. Therefore, in the coded excitation imaging, the received burst pulse is processed to a short pulse having a high amplitude through pulse compression, and a resolution in the axial direction increases. As a pulse compressor, a correlator, a Wiener filter, an inverse filter or the like may be used. Transmission power efficiency, which may be represented as a ratio of power of the ultrasound output from the transducer with respect to power of the transmitting pulse applied to the transducer for the coded excitation imaging, may increase. In order to increase transmission power efficiency, a frequency bandwidth of the transmitting pulse may match a limited bandwidth of the transducer. In order to generate the burst pulse having a frequency bandwidth that may match a bandwidth of the transducer, an arbitrary waveform may be generated. In order to generate the arbitrary waveform and apply the generated waveform to the transducer, a D/A converter and a high voltage power amplifier may be used. However, in this case, since the D/A converter and the high voltage power amplifier need to be mounted on all channels, the transmitter of the ultrasound probe may become a large system that requires high power so that it is complex and difficult to implement the ultrasound probe using a 2D array transducer and it is difficult to satisfy demand for miniaturization. Accordingly, an exemplary embodiment provides the transmitter that obtains the above effects that can be obtained when any waveform is transmitted through the modified pulse width modulation method and that may be implemented as a compact system requiring low power.

The ultrasound travels through a medium, has a waveform that is changed due to nonlinearity of the medium, and a harmonic component is accordingly generated. In the harmonic imaging, a harmonic component of the echo ultrasound generated due to nonlinearity of the medium may be used to obtain an image having a more improved resolution. The transducer such as the cMUT generates a non-linear frequency component due to its inherent characteristics when the ultrasound is transmitted, and generally generates, in particular, a harmonic component having a frequency 2f0 that is twice a transmission frequency f0. As described above, the harmonic imaging generally uses a harmonic component of the echo ultrasound, in particular, a harmonic component 2f0 that is twice the transmission frequency f0. Therefore, it is possible to remove a 2f0 component generated due to characteristics of the transducer such as the cMUT when the ultrasound is transmitted. Accordingly, in an exemplary embodiment, there is provided a transmitter in which a modulation signal generated by the modified pulse width modulation and a modulation signal that is shifted by a predetermined time from the modulation signal are synthesized, and a 2f0 component generated due to characteristics of the transducer may be removed. Hereinafter, transmit beamforming will be described and then the modified pulse width modulation will be described in detail.

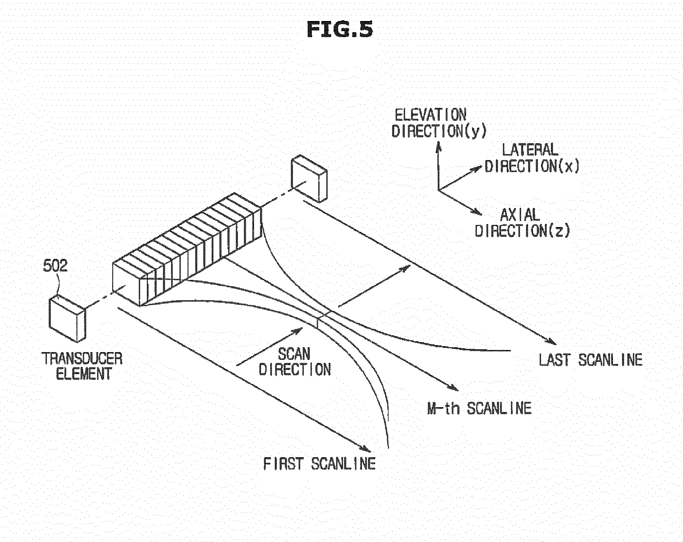

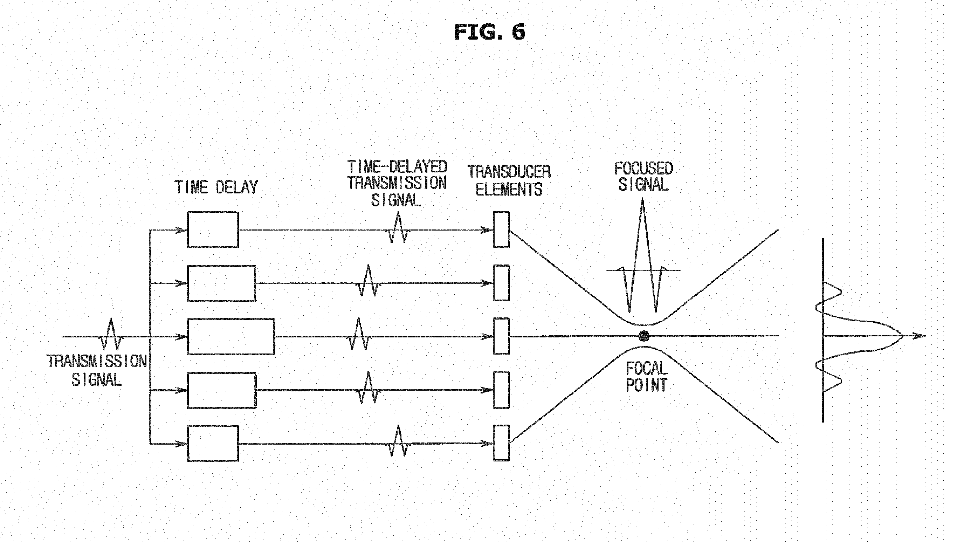

The transmission unit forms a transmission signal pattern according to a control signal of the controller of the main body and performs transmit beamforming. The transmission unit forms a transmission signal pattern based on a time delay value of each of the ultrasound transducer elements that constitute the 2D ultrasound transducer array and that is calculated through the controller. FIG. 5 is a diagram illustrating an operation of transmit beamforming using a 1D array transducer. FIG. 6 is a diagram illustrating a delay of a transmission signal applied when transmit beamforming is performed. In an exemplary embodiment, the 2D array transducer is used. However, for convenience of description, an example of the 1D array transducer is shown in FIG. 5, to describe transmit beamforming. As illustrated in FIG. 5, a 3D space in which ultrasound imaging is performed may be defined by a y axis corresponding to an elevation direction, an x axis corresponding to a lateral direction, and a z axis corresponding to an axial direction. A spatial resolution of a 2D ultrasound image may be determined by an axial direction resolution and a lateral direction resolution. The axial direction resolution refers to an ability to distinguish two objects arranged along an axis of an ultrasound beam. The lateral direction resolution refers to an ability to distinguish two objects that are perpendicularly arranged with respect to an axis of an ultrasound beam. The axial direction resolution is determined by a pulse width of the ultrasound signal to be transmitted. As the ultrasound signal has a shorter pulse width, in other words, as the ultrasound signal has a higher frequency in the same period, the axial direction resolution improves. The lateral direction resolution and the elevation direction resolution are determined according to a width of the ultrasound beam. As the ultrasound beam has a narrower width, the lateral direction resolution improves. In transmit beamforming, ultrasound signals transmitted from a plurality of transducer elements are focused at a focal point on a scan line, and thus an ultrasound beam having a narrow width may be formed. When the ultrasound beam having a narrow width is formed by transmit beamforming, a resolution of the ultrasound image, and particularly, the lateral direction resolution may be increased.

The 1D array transducer includes a plurality of transducer elements 502 that are one-dimensionally arranged. In order to obtain a 2D ultrasound cross-section image, a plurality of scan lines are needed. Beamforming with respect to the focal point may be performed from a first scan line to the last scan line. When the transducer array transmits the ultrasound signal to all scan lines, and receives an ultrasound echo signal that is reflected from an internal region of the object, the ultrasound diagnostic apparatus may obtain a 2D ultrasound cross-section image on an x-z plane. In order to focus the ultrasound beam at a point, ultrasound signals transmitted from the plurality of transducer elements should arrive at one focal point at the same time. As illustrated in FIG. 6, a distance between each of the elements and the focal point is different. Therefore, when an appropriate time delay is applied to the ultrasound signal transmitted from each of the elements, ultrasound beams may arrive at the same focal point at the same time. When ultrasound signals are transmitted from all elements toward the focal point at the same time, the ultrasound signal transmitted from an element that is closer to the focal point arrives at the focal point earlier, and the arrival of the ultrasound signal transmitted from an element more distant from the focal point is delayed. Therefore, as illustrated in FIG. 6, in consideration of the time delay from when a transmission signal is applied to the element, the transmission signal may be applied later to an element that is closer to the focal point, and the transmission signal may be applied earlier to an element more distant from the focal point. Here, the transmission signal refers to an electrical signal that is converted into an ultrasound signal in the transducer element.

In the present exemplary embodiment, a plurality of transmission elements are provided to generate a transmission signal pattern in which a delay time is applied to a plurality of transmission signals that correspond in number to the transducer elements of the transducer array.

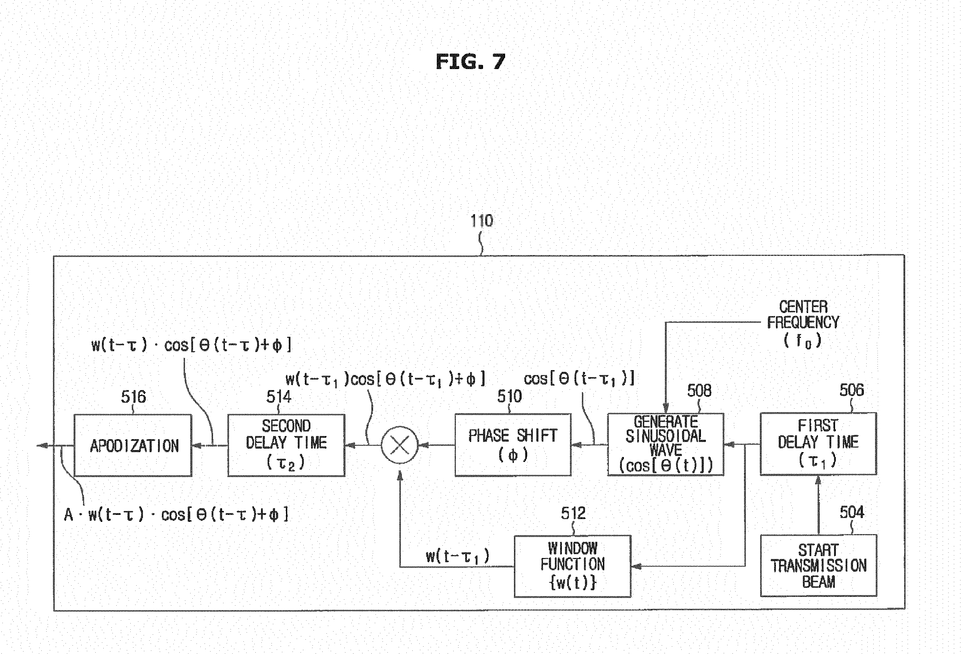

In order to perform the coded excitation imaging and the harmonic imaging, the transmission unit generates a waveform having a frequency bandwidth that may match a bandwidth of the transducer. For example, the transmission unit generates a sine or cosine signal to which a window function such as the Hanning window, the Hamming window, Gaussian window, or Kaiser window is applied, a chirp signal to which the window function is applied, a Golay code or the like. FIG. 7 is a diagram illustrating a process of generating a waveform in a transmission unit of an ultrasound probe according to an exemplary embodiment.

As illustrated in FIG. 7, when generation of a transmission beam starts (operation 504), the transmission unit applies a first delay time to a cosine signal having a center frequency of f0, and shifts the cosine signal to which the first delay time is applied by a predetermined phase (operations 506, 508, 510). The transmission unit applies the window function to the generated and shifted cosine signal (operation 512) and performs pulse shaping. The transmission unit applies a second delay time (operation 514) to the cosine signal to which the window function is applied, performs apodization (operation 516), and generates a transmission signal pattern. The apodization is a technique in which a different size is applied to each of the transducer elements of the transducer array, the window function is applied to an aperture in an x axis direction, and a side-lobe level of an x axis direction beam pattern is decreased.

While FIG. 7 illustrates a case in which the delay time is applied twice, this is only an example. At least one delay time may be applied and the delay times to be applied may be the same or different from each other.

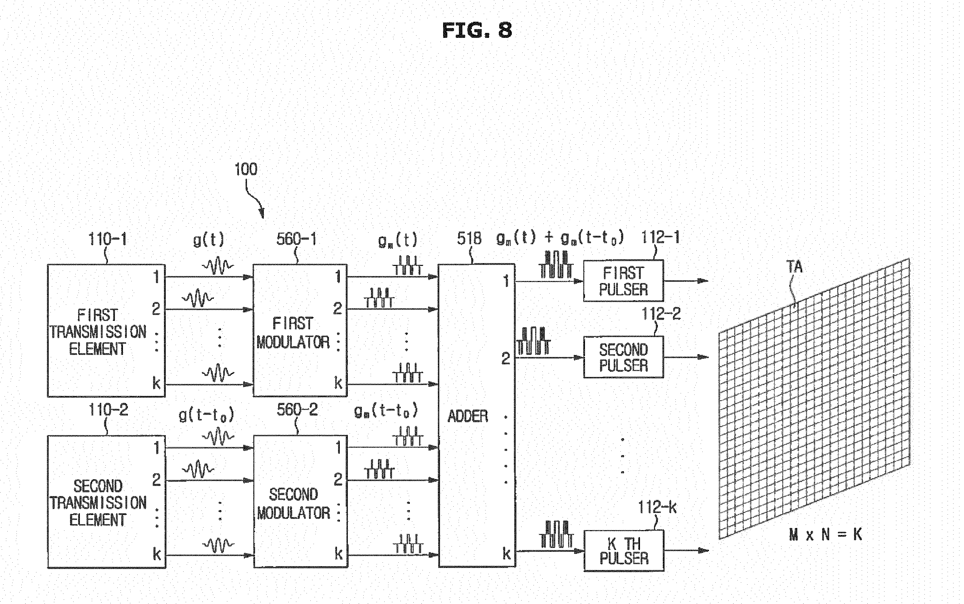

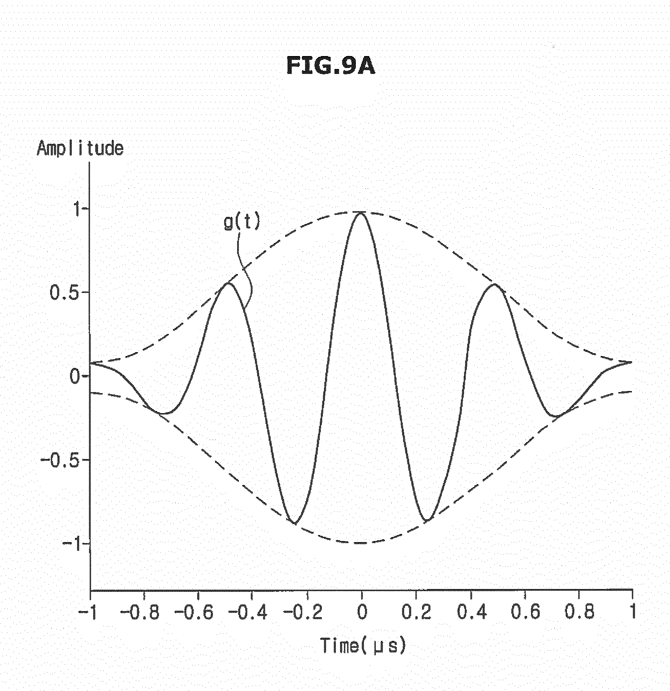

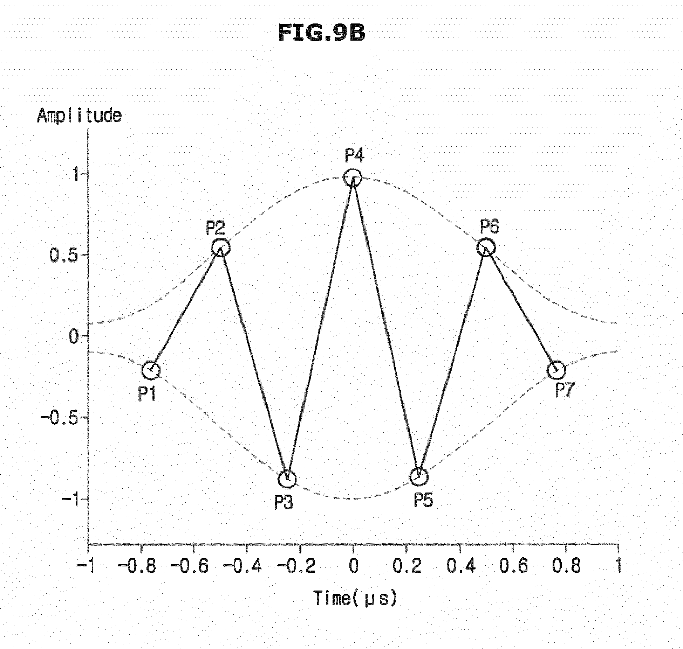

FIG. 8 is a diagram illustrating a process of modulating a transmission signal in a transmitter according to an exemplary embodiment. FIGS. 9A and 9B are diagrams illustrating waveforms of a transmission signal and a modulation signal generated in a transmitter according to an exemplary embodiment. Through the process illustrated in FIG. 7, a first transmission signal pattern and a second transmission signal pattern that are transmit beamformed and generated from the transmission elements, for example, a first transmission element 110-1 and a second transmission element 110-2 are input to a first modulator 560-1 and a second modulator 560-2, respectively. For example, as illustrated in FIG. 9A, the first transmission element and the second transmission element generate a transmission signal pattern formed of a cosine signal to which the window function is applied and output the pattern to each of the modulators. A transmission signal $g(t)$ illustrated in FIG. 9A is a cosine signal to which the window function is applied and has a center frequency of 2 MHz. The center frequency of the transmission signal $g(t)$ may be determined to match a frequency bandwidth of the transducer. FIG. 8 illustrates only two transmission elements for convenience of description, but an exemplary embodiment is not limited thereto. The present exemplary embodiment may include two or more transmission elements. In the present exemplary embodiment, the second transmission element 110-2 generates a second transmission signal $g(t-t0)$ that is shifted by a predetermined time t0 from a first transmission signal $g(t)$ generated in the first transmission element 110-1. Regulating of the time t0 to be shifted may be performed by regulating the delay time applied in the transmission element. As described above, the harmonic imaging generally uses the harmonic component of the echo ultrasound, and particularly, the harmonic component 2f0 that is twice the transmission frequency f0. Therefore, it is possible to remove a non-linear component including a 2f0 component generated due to characteristics of the transducer such as the cMUT when the ultrasound is transmitted. The transmission signal shifted by a predetermined time is generated using the plurality of transmission elements so that the 2f0 component generated due to such characteristics of the transducer is removed, as described in detail below.

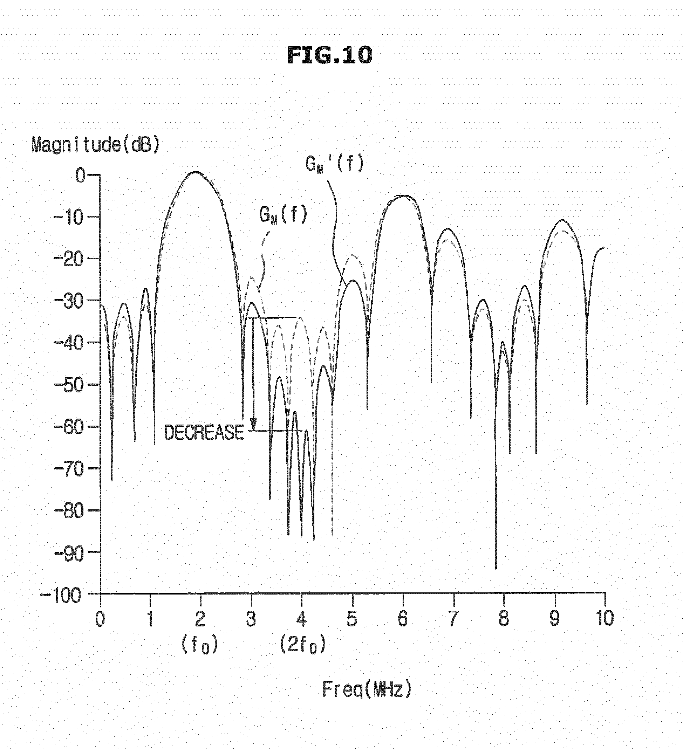

The modulator applies a pulse width modulation method to each transmission signal $g(t)$ forming the input transmission signal pattern and modulates the transmission signal $g(t)$. The modulator determines a peak level and a base level for each period of the transmission signal $g(t)$, and generates a modulation signal $gm(t)$ having a pulse width proportional to a size of an amplitude of the transmission signal $g(t)$ at the determined peak level and base level. FIG. 9B illustrates the modulation signal of the transmission signal $g(t)$ of the time domain illustrated in FIG. 9A. FIG. 10 illustrates a signal that is obtained by converting the modulation signal $gm(t)$ of the time domain into the frequency domain. As illustrated in FIG. 10, a signal $Gm(f)$ that is obtained by converting the modulation signal $gm(t)$ into the frequency domain has a similar center frequency band with a signal $(R)$ that is obtained by converting the transmission signal $g(t)$ into the frequency domain. In other words, it can be understood that the modulation signal $gm(t)$ very closely follows matching for the transducer of the transmission signal $g(t)$ that matches a frequency bandwidth with a limited bandwidth of the transducer to be suitable for the coded excitation imaging.

As illustrated in FIG. 8, the first modulator 560-1 modulates the first transmission signal pattern output from the first transmission element 110-1 through the above-described pulse width modulation method and outputs a first modulation signal $gm(t)$. The second modulator 560-2 outputs a second modulation signal $gm(t-t0)$ that is obtained by modulating the second transmission signal pattern output from the second transmission element 110-2 through the above-described pulse width modulation method. Since the second transmission signal pattern is shifted by a predetermined time t0 from the first transmission signal pattern, the second modulation signal $gm(t-t0)$ is also shifted by t0 from the first modulation signal $gm(t)$. The first modulation signal $gm(t)$ and the second modulation signal $gm(t-t0)$ that is shifted by t0 from the first modulation signal $gm(t)$ are synthesized in an adder 518. FIG. 10 illustrates a synthesized modulation signal $Gm'(t)$ that is obtained by converting the first modulation signal $gm(t)$ and the second modulation signal $gm(t-t0)$ that are synthesized by the adder into the frequency domain. FIG. 10 illustrates a frequency domain signal Gm'(t) of a synthesized modulation signal gm'(t) and a frequency domain signal Gm(f) of the first modulation signal gm(t) before it is synthesized with the time-shifted second modulation signal gm(t-t0) and illustrates an amount of difference of a harmonic component 4 MHz that is twice the center frequency 2 MHz before and after the time-shifted modulation signal is synthesized.

As illustrated in FIG. 10, a size of the synthesized modulation signal Gm'(f) is decreased by about 20 dB or more than a size of the modulation signal Gm(f) before synthesizing at 4 MHz that is twice the center frequency. As illustrated in FIG. 10, when the second modulation signal gm(t-t0) shifted by a predetermined time is added to the first modulation signal gm(t), it is possible to decrease the harmonic component that is twice the center frequency. Therefore, an ultrasound appropriate for the harmonic imaging may be output using the exemplary modified pulse width modulation method and the method of adding the time-shifted modulation signal according to an exemplary embodiment. A time to be shifted to decrease the harmonic component that is twice the center frequency should be determined. As illustrated in FIG. 10, a method of calculating a time to be shifted to decrease the harmonic component that is twice the center frequency will be described with reference to the following Equations.

<Equation 1>

$$g(t) \leftrightarrow G(f) = |G(f)| e^{\angle G(f)}$$

<Equation 2>

$$g'(t) = g(t) + g(t - t_0) \leftrightarrow G'(f) = G(f) + G(f) e^{-j2\pi f t_0}$$

$$G'(f) = G(f)\left(1 + e^{-j2\pi f t_0}\right)$$

<Equation 3>

$$|G'(f)| = |G(f)|\left|1 + e^{-j2\pi f t_0}\right|$$

<Equation 4>

$$\left|1 + e^{-j2\pi f t_0}\right| = \sqrt{\left(1 + \cos 2\pi f t_0\right)^2 + \sin^2 2\pi f t_0} = \sqrt{2\left(1 + \cos 2\pi f t_0\right)}$$

$$= \sqrt{4\left(\frac{1 + \cos 2\pi f t_0}{2}\right)} = 2\sqrt{\cos^2 \pi f t_0} = 2\left|\cos \pi f t_0\right|$$

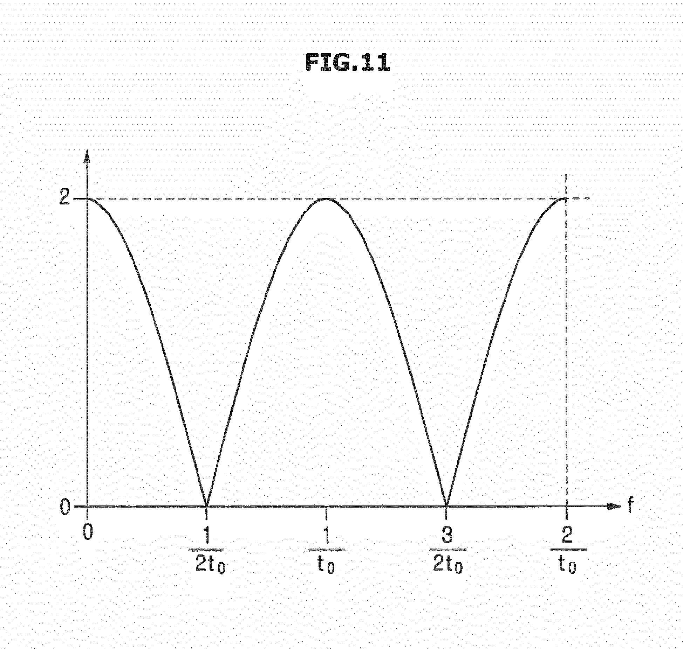

In Equation 1, g(t) denotes a transmission signal, and G(f) denotes a signal that is obtained by converting g(t) into the frequency domain. In Equation 2, g(t-t0) denotes a signal that is obtained by shifting g(t) by t0, g'(t) denotes a signal that is obtained by adding g(t) and g(t-t0), and G'(f) denotes a signal that is obtained by converting g'(t) into the frequency domain and t0 denotes a shift time to be obtained. Equation 3 denotes an absolute value of G'(f). Equation 4 simplifies Equation 3 except for a part denoting an amplitude in the form of a cosine signal. The cosine signal in Equation 4 has a form in which an absolute value of the cosine signal having a period of 2/t0 is obtained as illustrated in FIG. 11. The signal illustrated in FIG. 11 has a zero size at 1/2t0 for the first time, t0 at which 1/2t0 becomes 2f0 that is the harmonic component of twice a center frequency f0 of the transmission signal is calculated and applied, and thus twice the harmonic component of G'(f) may be decreased as illustrated in FIG. 10. Also, t0 that satisfies 1/2t0 = 2f0 becomes TO/4, (T0 = 1/f0). In other words, when the second modulation signal gm(t-t0) that is a modulation signal of the transmission signal obtained by shifting a time corresponding to 1/4 of a period of the transmission signal is added to the first modulation signal gm(t), it is possible to obtain an effect of decreasing the harmonic component that is twice the center frequency of the synthesized modulation signal gm'(t) as illustrated in FIG. 10.

As illustrated in FIG. 8, the synthesized modulation signal gm'(t) in which the first modulation signal gm(t) and the second modulation signal gm(t-t0) are synthesized by the adder 518 is input to the first pulser 112-1 and the second through Kth pulsers 112-2 through 112-k which are equal in number K to the transducer elements of the transducer array. The pulser outputs the transmitting pulse corresponding to the synthesized modulation signal gm'(t) and applies the output to the transducer element. Although not illustrated, the transmitter may further include a memory in which information on the synthesized modulation signal gm'(t) is stored. The pulser may be implemented as an on-off pulser, for example, a bi-polar pulser or a uni-polar pulser. The memory may encode a waveform of the synthesized modulation signal gm'(t), for example, to 1, 0, or -1, and store the result. According to information on the modulation signal stored in the memory, the pulser outputs the transmitting pulse and applies the output to each of the transducer elements. The transmitter may further include a low pass filter using the transmitting pulse as an input signal such that the transmitting pulse may match a frequency band of the transducer element more accurately. The low pass filter may be implemented to pass the center frequency band of the transmission signal and block the harmonic component that is twice the center frequency.

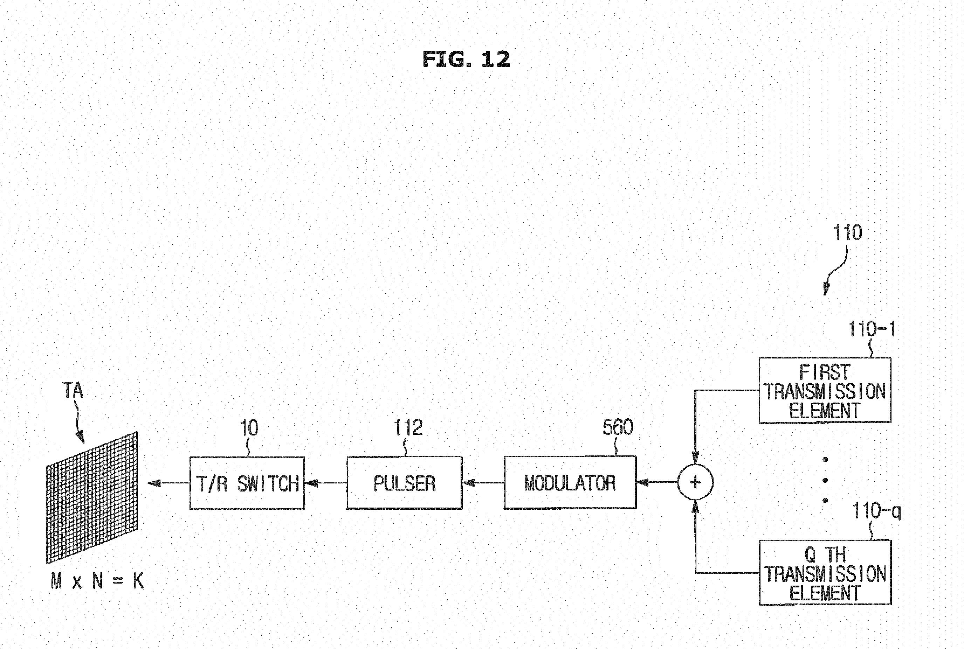

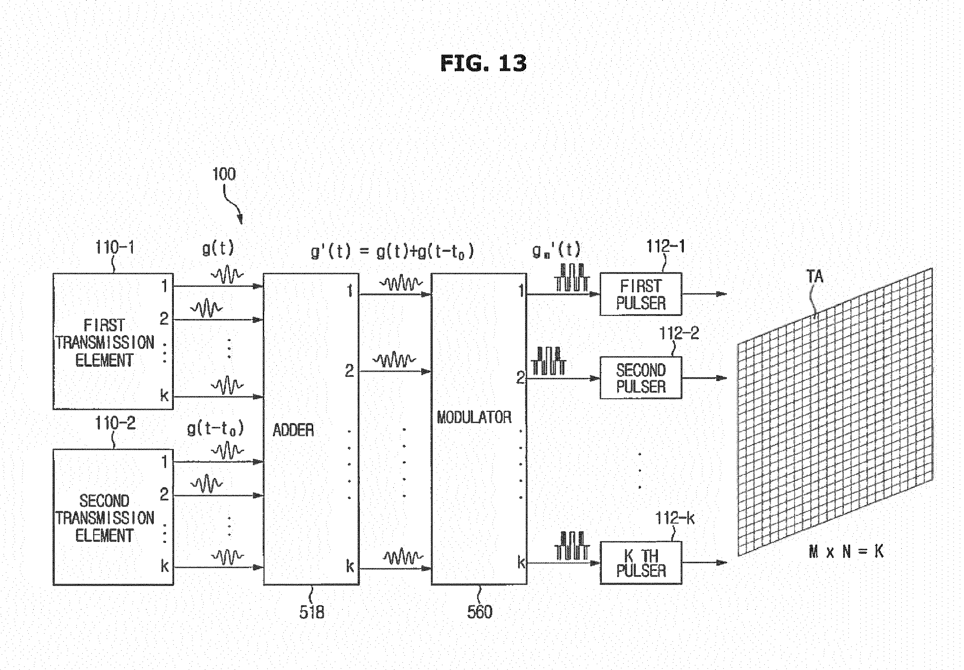

FIG. 12 is a diagram illustrating a configuration of a transmitter according to an exemplary embodiment. FIG. 13 is a diagram illustrating modulation of a transmission signal in a transmitter according to an exemplary embodiment. As illustrated in FIGS. 12 and 13, the transmitter includes a plurality of transmission elements, a modulator and a pulser.

Similar to the above-described, in the present exemplary embodiment, a plurality of the transmission elements configured to generate the above-described transmission signal pattern are provided. The plurality of transmission elements generate a transmission signal pattern in which a delay time is applied to a plurality of transmission signals which correspond in number to the elements of the transducer array. In order to perform the coded excitation imaging and the harmonic imaging, the transmission unit generates a waveform having a frequency bandwidth that may match a bandwidth of the transducer. For example, the transmission unit generates a cosine signal to which the window function is applied, a chirp signal to which the window function is applied, a Golay code or the like. Since the process of the transmission unit generating the transmission signal pattern formed of the cosine signal to which the window function is applied is described above with reference to FIG. 7, the process of the transmission unit generating a waveform will not be described.

In the present exemplary embodiment, the first transmission signal pattern and the second transmission signal pattern that are transmit beamformed and generated by the transmission elements, for example, the first transmission element 110-1 and the second transmission element 110-2 are not input to the first modulator and the second modulator, but are synthesized. The present exemplary embodiment includes one modulator configured to modulate a synthesized transmission signal in which transmission signal patterns generated from the transmission elements are synthesized.

The first transmission element and the second transmission element generate a transmission signal, for example, a transmission signal pattern formed of a cosine signal to which the window function is applied as illustrated in FIG. 9A. As described above, the transmission signal is a cosine signal to which the window function is applied and has a center frequency of 2 MHz. The center frequency of the transmission signal may be determined to match a frequency bandwidth of the transducer element. FIG. 13 illustrates only two transmission elements 110-1 and 110-2 for convenience of description, but the present exemplary embodiment is not limited thereto. The present exemplary embodiment may include two or more transmission elements. In the present exemplary embodiment, the second transmission element 110-2 generates a second transmission signal g(t-t0) that is shifted by a predetermined time t0 from a first transmission signal g(t) generated in the first transmission element 110-1. Regulating of the time t0 to be shifted may be performed by regulating the delay time applied in the transmission element. As described above, the harmonic imaging generally uses the harmonic component of the echo ultrasound, and particularly, the harmonic component 2fD that is twice the transmission frequency f0. Therefore, it is possible to remove a non-linear component including a 2f0 component generated due to such characteristics of the transducer such as the cMUT when the ultrasound is transmitted. The transmission signal shifted by a predetermined time is generated using the plurality of transmission elements so that the 2f0 component generated due to such characteristics of the transducer is removed. Since this is the same as in the above-described exemplary embodiment, details thereof will be omitted. The first transmission signal pattern generated in the first transmission element and the second transmission signal pattern that is shifted by a predetermined time from the first transmission signal pattern and is generated in the second transmission element are synthesized in the adder 518. The synthesized transmission signal pattern is input to the modulator 560.

The modulator applies a pulse width modulation method to each transmission signal g'(t) forming the input synthesized transmission signal pattern and modulates the transmission signal. A principle of pulse width modulation according to the present exemplary embodiment will be described with reference to FIGS. 9A and 9B. The modulator determines a peak level and a base level for each period of a synthesized transmission signal g'(t), and generates a modulation signal gm'(t) having a pulse width proportional to a size of an amplitude of the synthesized transmission signal g'(t) at the determined peak level and base level. Since descriptions are the same as in FIGS. 9A and 9B of the above-described exemplary embodiment, details thereof will be omitted. The synthesized modulation signal gm'(t) that is generated by applying pulse width modulation to the synthesized transmission signal g'(t) by the modulator has a frequency spectrum Gm'(f) illustrated in FIG. 10. Unlike the above-described exemplary embodiment, in the present exemplary embodiment, without generating the synthesized modulation signal by synthesizing modulated signals, the synthesized transmission signal is modulated to generate the synthesized modulation signal, but a signal of the frequency domain of the generated modulation signal may also have a similar waveform to the signal illustrated in FIG. 10. As illustrated in FIG. 10, a size of the modulation signal Gm'(f) is decreased at 4 MHz that is twice the center frequency by about 20 dB or more compared to size of the modulation signal Gm(f) before synthesizing according to the above-described exemplary embodiment. As illustrated in FIG. 10, when the second transmission signal pattern shifted by a predetermined time is added to the first transmission signal pattern, it is possible to decrease the harmonic component that is twice the center frequency. Therefore, an ultrasound appropriate for the coded excitation imaging and the harmonic imaging may be output using the modified pulse width modulation method and the method of adding the time-shifted transmission signal according to the present exemplary embodiment. A time to be shifted to decrease the harmonic component that is twice the center frequency may be determined as described above.

As illustrated in FIG. 13, the synthesized modulation signal gm'(t) output from the modulator is input to the first and second through Kth pulsers 112-1 and 112-2 to 112-k, which are equal in number to the transducer elements of the transducer array. The pulser outputs the transmitting pulse corresponding to the synthesized modulation signal gm'(t) and applies the output to the transducer element. Although not illustrated, the transmitter may further include a memory in which information on the synthesized modulation signal is stored. The memory may encode a waveform of the synthesized modulation signal, for example, to 1, 0, or -1 and store the result. The pulser may be implemented as an on-off pulser, for example, a bi-polar pulser or a uni-polar pulser. According to information on the modulation signal stored in the memory, the pulser outputs the transmitting pulse and applies the output to each of the transducer elements. The transmitter may further include a low pass filter using the transmitting pulse as an input signal such that the transmitting pulse may match a frequency band of the transducer element more accurately. The low pass filter may be implemented to pass the center frequency band of the transmission signal and block the harmonic component that is twice the center frequency.

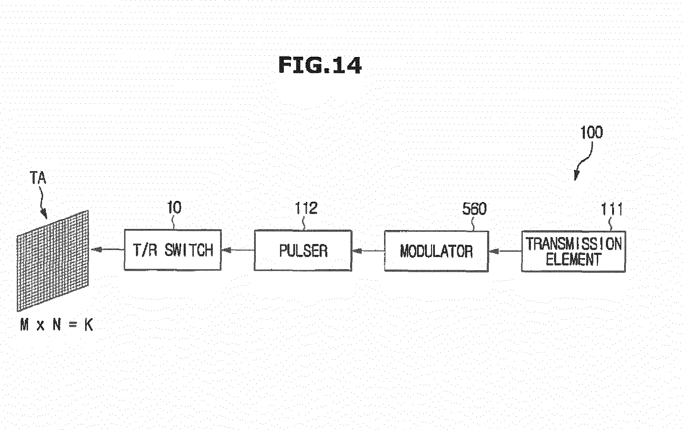

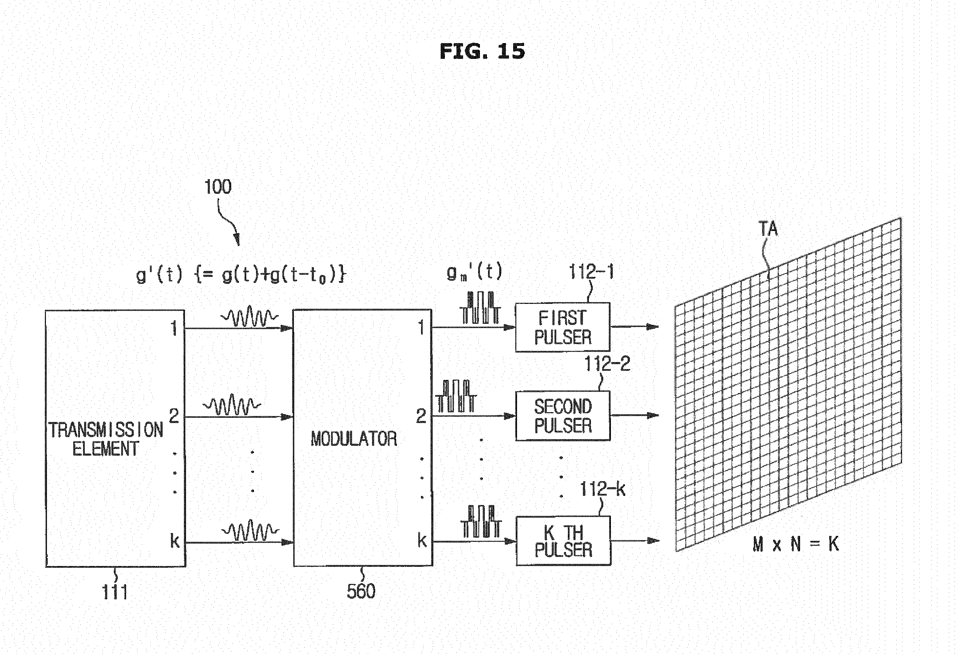

FIG. 14 is a diagram illustrating a configuration of a transmitter according to an exemplary embodiment. FIG. 15 is a diagram illustrating modulation of a transmission signal in a transmitter according to an exemplary embodiment. As illustrated in FIGS. 14 and 15, the transmitter includes a transmission unit including a single transmission element 111, a modulator and a pulser.

Unlike the above-described exemplary embodiment in which there are the plurality of transmission elements configured to generate a transmission signal pattern, one transmission element is provided in the present exemplary embodiment. The transmission element generates a transmission signal pattern to which a delay time is applied to the plurality of transmission signals that correspond in number to the transducer elements of the transducer array. In order to perform the coded excitation imaging and the harmonic imaging, the transmission element generates a waveform having a frequency bandwidth that may match a bandwidth of the transducer. For example, the transmission element generates a cosine signal to which the window function is applied, a chirp signal to which the window function is applied, a Golay code or the like. Since the process of the transmission element generating the transmission signal pattern formed of the cosine signal to which the window function is applied is described above with reference to FIG. 7, the process of the transmission element generating a waveform will not be described.

The present exemplary embodiment includes one transmission element, and one transmission element generates a synthesized transmission signal pattern in which the first transmission signal pattern generated and the second transmission signal pattern are synthesized. As described above, it is possible to generate directly the synthesized transmission signal pattern by regulating the delay time applied to the transmission signal. The synthesized transmission signal pattern generated in the transmission element 111 is input to the modulator 560. The modulator applies a pulse width modulation method to each transmission signal g'(t) forming the input synthesized transmission signal pattern and modulates the transmission signal. Since descriptions are the same as in FIGS. 9A and 9B, details thereof will be omitted.

The synthesized modulation signal gm'(t) that is generated by applying pulse width modulation to the synthesized transmission signal g'(t) by the modulator has a frequency spectrum Gm'(f) illustrated in FIG. 10. In the present exemplary embodiment, without generating the synthesized modulation signal by synthesizing modulated signals, the synthesized transmission signal is modulated to generate the synthesized modulation signal, but a signal of the frequency domain of the generated modulation signal may have a similar waveform to the signal illustrated in FIG. 10.

As illustrated in FIG. 15, the synthesized modulation signal gm'(t) output from the modulator 560 is input to the first and second to Kth pulsers, which are equal in number to the transducer elements of the transducer array. The pulser outputs the transmitting pulse corresponding to the synthesized modulation signal gm'(t) and applies the output to the transducer element. Although not illustrated, the transmitter may further include a memory in which information on the synthesized modulation signal gm'(t) is stored. Since other details are the same as in FIGS. 8 and 13, descriptions thereof will be omitted.

In exemplary embodiments, through the modified pulse width modulation method and the method of adding the time-shifted transmission signal or modulation signal, the transmitting pulse that may match a frequency band of the transducer element may be applied to the transducer element, and the harmonic component that is twice the center frequency of the transmission signal may be removed. Therefore, the ultrasound probe including the transmitter according to exemplary embodiments may output an ultrasound appropriate for both the coded excitation imaging and the harmonic imaging.

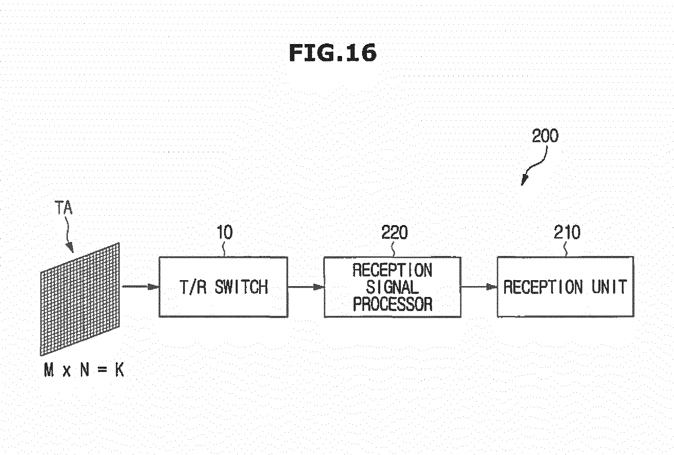

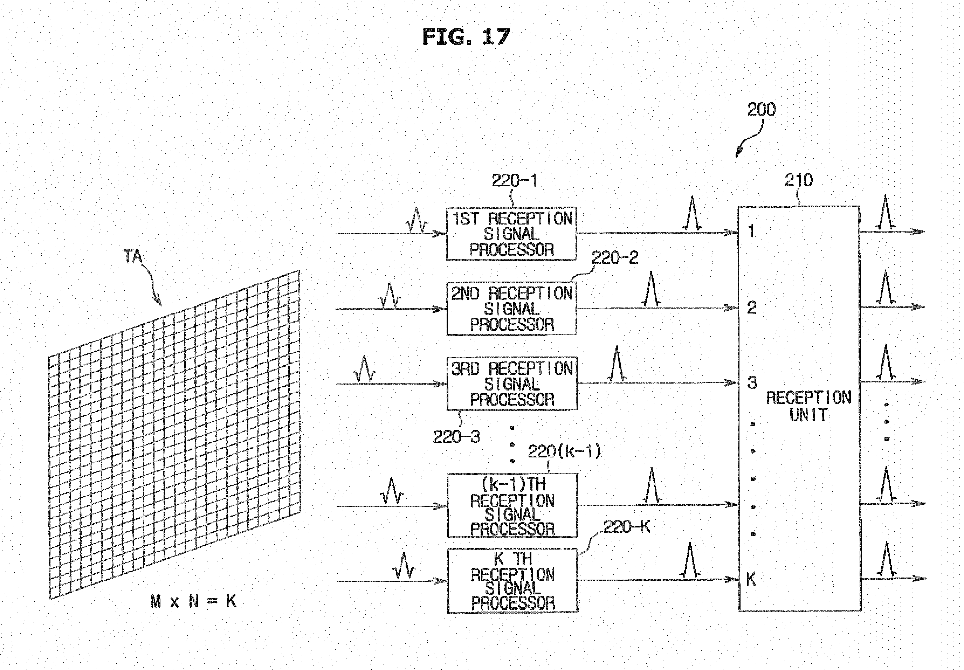

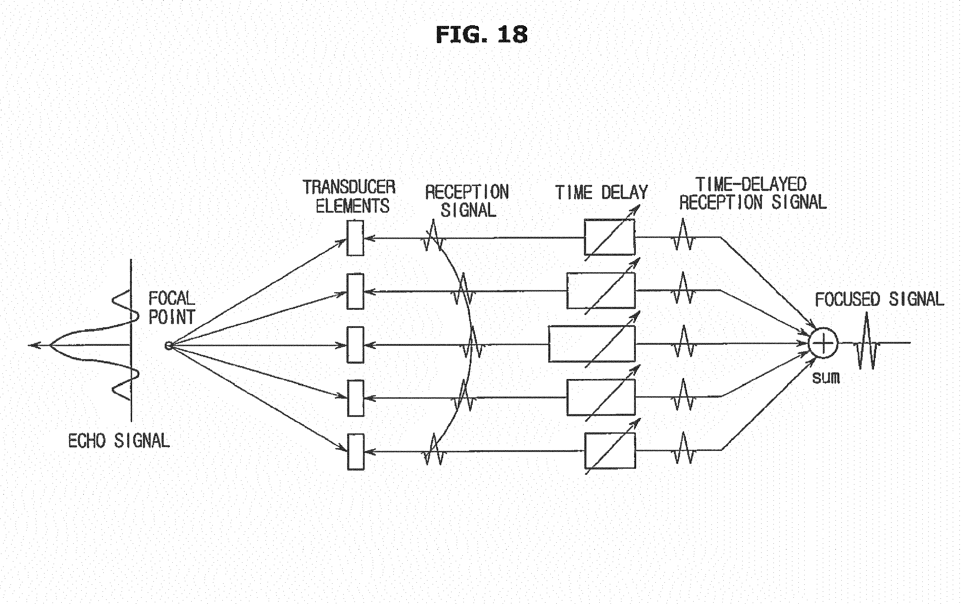

FIG. 16 is a diagram illustrating a configuration of a receiver according to an exemplary embodiment. FIG. 17 is a diagram illustrating receive beamforming in a receiver according to an exemplary embodiment. FIG. 18 is a diagram illustrating a delay of a reception signal applied when receive beamforming is performed. The receiver of the ultrasound probe according to an exemplary embodiment performs a predetermined process on an ultrasound echo signal received in the transducer array and performs receive beamforming.

As illustrated in FIG. 16, the receiver 200 includes a reception signal processor 220 and a reception unit 210.

The ultrasound echo signal that is reflected from the focal point and returned is input to the transducer array. The transducer array converts the input ultrasound echo signal into an analog electrical signal (hereinafter referred to as an electrical signal).

As illustrated in FIG. 17, the electrical signal converted in the transducer array is input to the reception signal processor 220 including a first reception signal processor 220-1, a second reception signal processor 220-2, a third reception signal processor 220-3 through (K-1)th reception signal processor 220(k-1), and Kth reception signal processor 220-k. The reception signal processor may amplify a signal before signal processing or time delay processing is performed on the electrical signal obtained by converting the ultrasound echo signal, regulate a gain, or compensate for attenuation according to a depth. More specifically, the reception signal processor may include a low noise amplifier (LNA) configured to decrease noise of the electrical signal input from the ultrasound transducer array and a variable gain amplifier (VGA) configured to control a gain value according to the input signal. The variable gain amplifier may utilize time gain compensation (TGC) in which a gain according to a distance from the focal point is compensated for, but an exemplary embodiment is not limited thereto. The reception signal processor may include the pulse compressor configured to process the received burst pulse for the coded excitation imaging to a short pulse having a high amplitude through pulse compression. As the pulse compressor, a correlator, a Wiener filter, an inverse filter or the like may be used. As described above, the coded excitation imaging is an imaging method in which a degree of penetration and a resolution of the ultrasound are increased, and a clear image of a target area in a deep region of the object may be obtained. In order to increase a degree of penetration of the ultrasound, an output of the ultrasound should be increased. In the coded excitation imaging, in order to increase an output of the ultrasound, a burst pulse formed of a plurality of pulses rather than a single pulse is output. When the burst pulse is output, a degree of penetration of the ultrasound may increase, but a resolution in the axial direction decreases. Therefore, the reception signal processor may include the pulse compressor configured to process the burst pulse received in a coded excitation imaging mode to a short pulse having a high amplitude through pulse compression and that can increase a resolution in the axial direction.

The reception unit performs beamforming on the electrical signal input from the reception signal processor. The reception unit increases a strength of the signal using a method in which the electrical signals input from the reception signal processor are superimposed.

FIG. 18 is a diagram illustrating a delay of a reception signal applied when receive beamforming is performed. When the transmission unit performs transmit beamforming, and thus the ultrasound having the same phase arrives at the focal point, the ultrasound echo signal is generated from the focal point and is returned to the transducer array. Similar to transmitting the ultrasound to the focal point, when the ultrasound echo signal is received from the focal point, since a distance from the focal point differs for each transducer element, a time at which the ultrasound echo signal arrives becomes different. Specifically, the ultrasound echo signal arrives at an element that is the closer to the focal point earlier, and the ultrasound echo signal arrives at an element that is more distant from the focal point later. Since the ultrasound echo signal has a very small size, it is difficult to obtain necessary information using only one signal received in each of the elements. Similar to the transmit beamforming, in the receive beamforming, an appropriate delay time is applied to reception signals that arrive at the elements with a time difference and the signals are summed at the same time, thereby increasing a signal-to-noise ratio.

The signal that is beamformed in the reception unit is converted into a digital signal by an analog to digital converter (ADC), and transmitted to the image processor of the main body 8. When the ADC is provided in the main body, the analog signal beamformed in the reception unit is transmitted to the main body and the main body may convert the analog signal into the digital signal. The reception unit may be a digital beamformer. The digital beamformer may include a memory in which the analog signal may be sampled and stored, a sampling period controller capable of controlling a sampling period, an amplifier capable of regulating a size of a sample, an anti-aliasing low pass filter configured to prevent aliasing before sampling, a bandpass filter capable of selecting a desired frequency band, an interpolation filter capable of increasing a sampling rate when beamforming is performed, a high-pass filter capable of removing a DC component or a signal of a low frequency band and the like.

When the 2D array transducer is used, the number of channels may be substantially increased, and thus system implementation may become complicated. When the process may pass through the reception unit a plurality of times in order to prevent such a problem, the number of channels may decrease. Since signals output from the reception unit are outputs of input signals that are summed, the number of signals output from the reception unit is smaller than the number of input signals. Therefore, when the process passes through the reception unit several times, it is possible to decrease the number of channels.

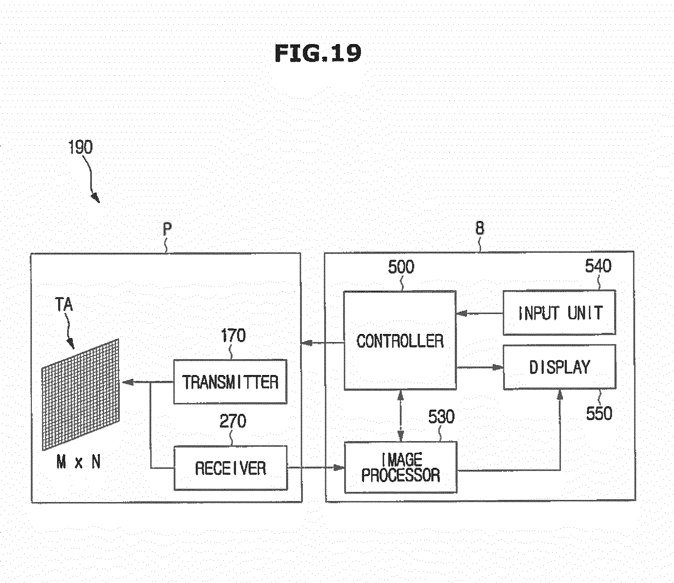

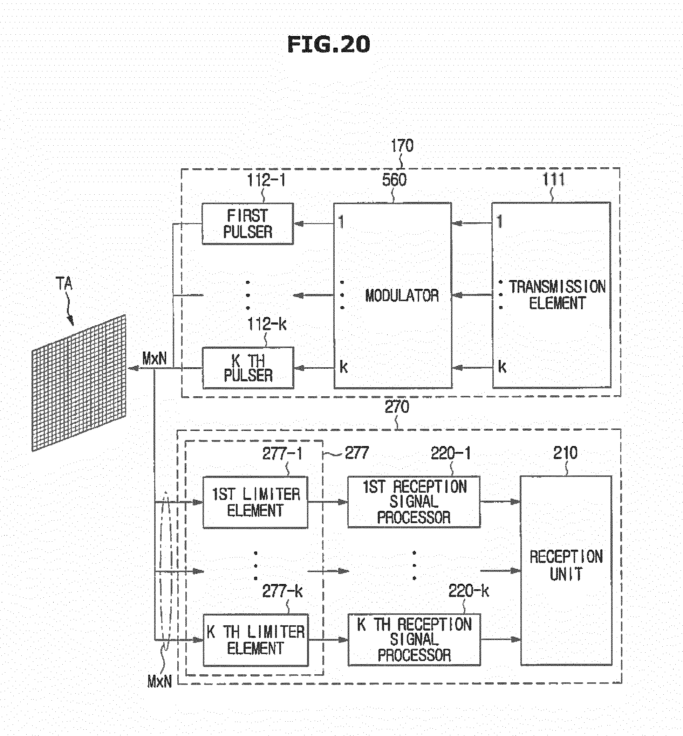

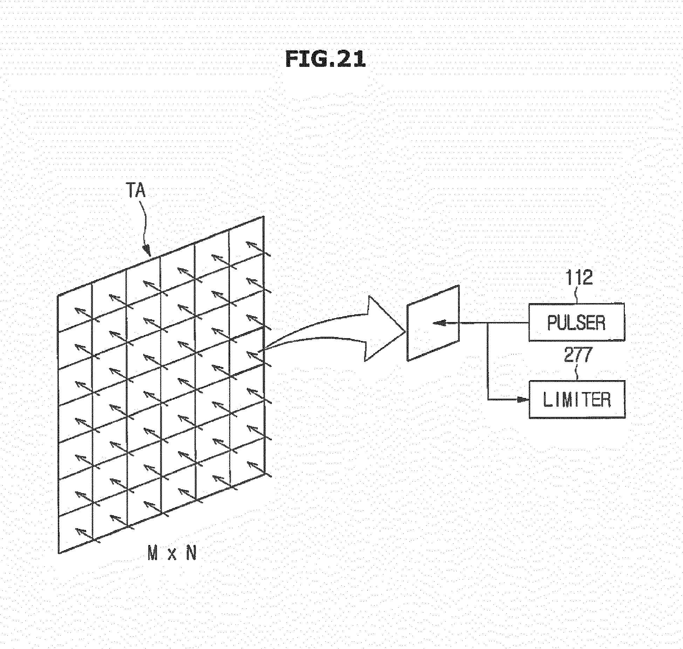

FIG. 19 is a block diagram illustrating a configuration of an ultrasound diagnostic apparatus 190 according to an exemplary embodiment. FIGS. 20 and 21 are diagrams illustrating a detailed configuration of a transmitter and a receiver of an ultrasound probe according to an exemplary embodiment. Since the main body of the ultrasound diagnostic apparatus according to the present exemplary embodiment has the same configuration as the main body as described above, the description thereof will be omitted. The ultrasound probe according to the present exemplary embodiment includes a transducer array TA, a transmitter 170, and a receiver 270.

Since a transmission element 111, a modulator 560 and a pulser 112 included in the transmitter 170 are the same as described above, the redundant descriptions will be omitted. Also, FIG. 20 illustrates one transmission element and one modulator. However, similar to the above-described exemplary embodiments, a plurality of transmission elements and a plurality of modulators may be provided. The ultrasound probe according to the present exemplary embodiment has no T/R switch configured to regulate a transceiving state. Since the ultrasound probe according to the present exemplary embodiment has no T/R switch, even when the state is in a transmitting state in which the pulser applies a transmitting pulse of a high voltage, a connection between a reception circuit and a transmission circuit including a pulser is not blocked. When the reception circuit and the transmission circuit are connected in a transmitting state, a high voltage applied from the transmission circuit in a transmitting state is also applied to the reception circuit, and the reception circuit may fail. Therefore, in order to prevent the reception circuit from failing due to the high voltage applied from the transmission circuit in a transmitting state, the ultrasound probe according to the present exemplary embodiment includes a limiter 277 that is connected to the transmission circuit including the pulser and is able to block a high voltage, as illustrated in FIG. 20. The limiter 277 may include first through Kth limiter elements 277-1 through 277-k. The limiter is an amplitude limiter that uses a diode and limits an upper limit of a voltage, and may be implemented in the form in which a peak clipper and a base clipper are combined. As illustrated in FIGS. 20 and 21, similar to the pulser, the limiter is connected to all M x N elements, blocks a high voltage generated in a transmitting state, and thus protects the receiver. FIG. 21 illustrates a case in which the pulser and the limiter are connected to each of the elements. Since the ultrasound probe according to the present exemplary embodiment does not include the T/R switch, it may be advantageous in terms of a manufacturing cost or implementation of the ultrasound probe. The electrical signal converted in the transducer array passes through the limiter and a reception signal processor 220 connected to each of the transducer elements. The reception signal processor may include a preamplifier capable of amplifying an electrical signal, and other components as described above. The electrical signal that has passed through the reception signal processor is input to a reception unit 210. The reception unit performs beamforming on the electrical signal input from the reception signal processor. Since descriptions of the reception unit and the receive beamforming are the same as described above, the redundant descriptions will be omitted.

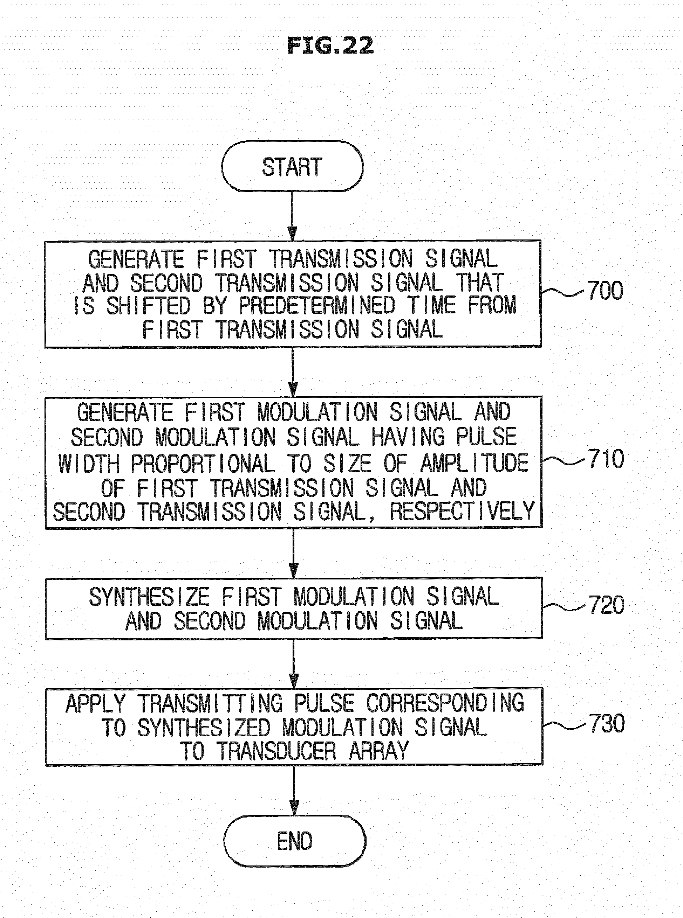

FIG. 22 is a flowchart illustrating a method of generating an ultrasound signal according to an exemplary embodiment.

As illustrated in FIG. 22, the transmission element generates a first transmission signal and a second transmission signal that is shifted by a predetermined time from the first transmission signal (operation 700).

In the present exemplary embodiment, the transmission elements generate a transmission signal pattern in which a delay time is applied to a plurality of transmission signals that correspond in number to elements of the transducer array.

The present exemplary embodiment may include two or more transmission elements. In the present exemplary embodiment, the second transmission element generates a second transmission signal g(t-t0) that is shifted by a predetermined time t0 from a first transmission signal g(t) generated in the first transmission element. Regulating of the time to be shifted may be performed by regulating the delay time applied in the transmission element. The first transmission element generates the first transmission signal pattern obtained by applying the delay time to the first transmission signal g(t) and transmits the pattern to the first modulator. The second transmission element generates the second transmission signal pattern obtained by applying the delay time to the second transmission signal g(t-t0) that is shifted by t0/4 from the first transmission signal g(t) and transmits the pattern to the second modulator.

The modulator generates the first modulation signal and the second modulation signal having a pulse width proportional to a size of an amplitude of the first transmission signal and the second transmission signal, respectively (operation 710). The adder synthesizes the first modulation signal and the second modulation signal (operation 720).

The modulator applies a pulse width modulation method to each of the transmission signals of the input transmission signal pattern and modulates the transmission signal. The modulator determines a peak level and a base level for each period of the transmission signal, and generates a modulation signal having a pulse width proportional to a size of an amplitude of the transmission signal at the determined peak level and base level. As illustrated in FIG. 8, the first modulator modulates the first transmission signal pattern output from the first transmission element through the above-described pulse width modulation method and outputs a first modulation signal gm(t). The second modulator also outputs a second modulation signal gm(t-t0) that is obtained by modulating the second transmission signal pattern output from the second transmission element through the above-described pulse width modulation method. As described above, since the second transmission signal pattern is shifted by a predetermined time t0 from the first transmission signal pattern, the second modulation signal gm(t-t0) is also shifted by t0 from the first modulation signal gm(t). The first modulation signal gm(t) and the second modulation signal gm(t-t0) that is shifted by t0 from the first modulation signal gm(t) are synthesized in the adder. As illustrated in FIG. 10, when the second modulation signal gm(t-t0) shifted by a predetermined time is added to the first modulation signal gm(t), it is possible to decrease the harmonic component that is twice the center frequency. Therefore, an ultrasound appropriate for the harmonic imaging may be output using the modified pulse width modulation method and the method of adding the time-shifted modulation signal according to an exemplary embodiment.

The pulser applies the transmitting pulse corresponding to the synthesized modulation signal to the transducer array (operation 730).

As illustrated in FIG. 8, the synthesized modulation signal in which the first modulation signal gm(t) and the second modulation signal (gm(t-t0)) are synthesized is input to the pulsers, which are equal in number to the elements of the transducer array. The pulser outputs the transmitting pulse corresponding to the synthesized modulation signal and applies the output to the transducer element.

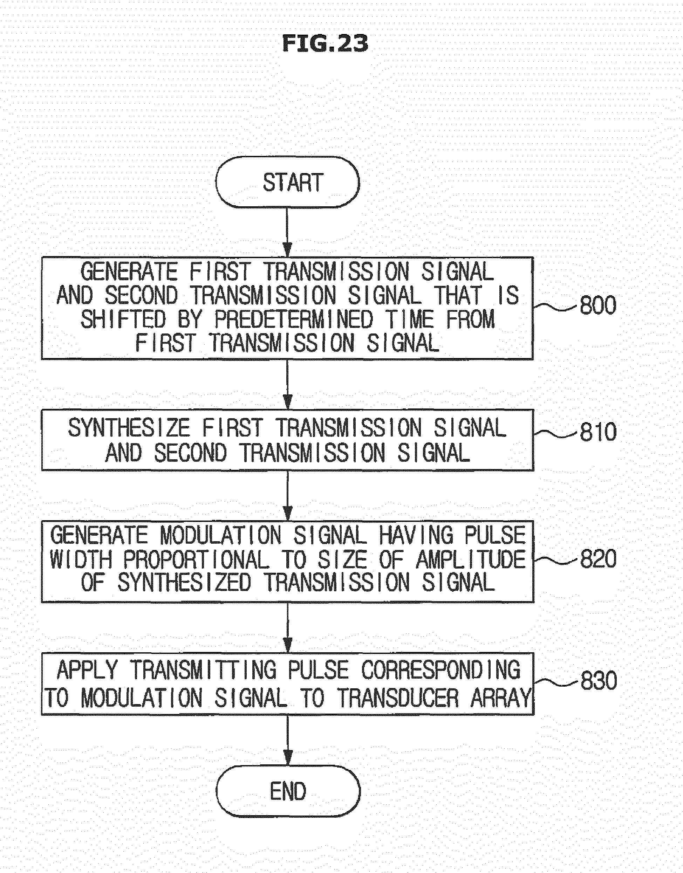

FIG. 23 is a flowchart illustrating a method of generating an ultrasound signal according to an exemplary embodiment. As illustrated in FIG. 23, the transmission unit generates a first transmission signal and a second transmission signal that is shifted by a predetermined time from the first transmission signal (operation 800), and the adder synthesizes the first transmission signal and the second transmission signal (operation 810).

Through the process illustrated in FIG. 7, the first transmission signal pattern and the second transmission signal pattern that are transmit beamformed and generated from the transmission elements, for example, the first transmission element and the second transmission element are synthesized. The present embodiment includes one modulator configured to modulate a synthesized transmission signal in which transmission signal patterns generated from the transmission elements are synthesized. The first transmission element and the second transmission element generate a transmission signal, for example, a transmission signal pattern formed of a cosine signal to which the window function is applied as illustrated in FIG. 9A. The present exemplary embodiment may include two or more transmission elements. In the present exemplary embodiment, the second transmission element generates a second transmission signal g(t-t0) that is shifted by a predetermined time t0 from a first transmission signal generated in the first transmission element. The second transmission element generates the second transmission signal pattern obtained by applying the delay time to the second transmission signal g(t-t0) that is shifted by t0/4 from the first transmission signal g(t). The adder synthesizes the first transmission signal pattern and the second transmission signal pattern and transmits the result to the modulator.

The modulator generates a modulation signal having a pulse width proportional to a size of an amplitude of the synthesized transmission signal (operation 820).

The modulator applies a pulse width modulation method to each of the transmission signals of the input synthesized transmission signal pattern and modulates the transmission signal, as described above. The pulser applies the transmitting pulse corresponding to the modulation signal to the transducer array (operation 830).

As illustrated in FIG. 13, the synthesized modulation signal output from the modulator is input to the pulsers, which are equal in number to the elements of the transducer array.

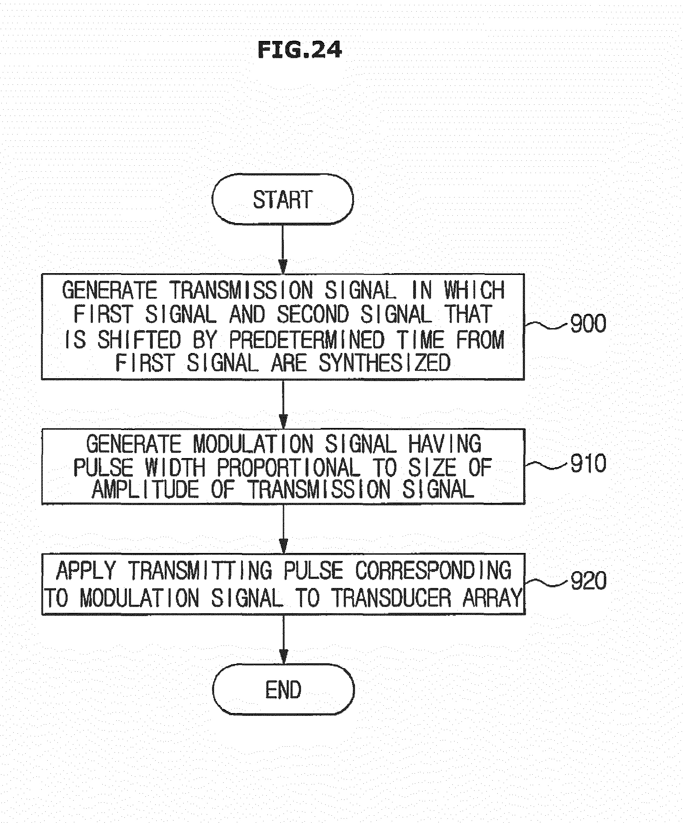

FIG. 24 is a flowchart illustrating a method of generating an ultrasound signal according to an exemplary embodiment. As illustrated in FIG. 24, the single transmission element generates a transmission signal in which a first transmission signal and a second signal that is shifted by a predetermined time from the first transmission signal are synthesized (operation 900).

The transmission element generates a transmission signal pattern to which a delay time is applied to the plurality of transmission signals that correspond in number to elements of the transducer array. The present exemplary embodiment includes one transmission element, and one transmission element generates a synthesized transmission signal pattern. As described above, since the transmission element may generate a waveform, it is possible to generate directly the synthesized transmission signal pattern by regulating the delay time applied to the transmission signal. The transmission element transmits the generated transmission signal pattern to the modulator.

The modulator generates a modulation signal having a pulse width proportional to a size of an amplitude of the transmission signal (operation 910).

The modulator applies a pulse width modulation method to each of the transmission signals of the input synthesized transmission signal pattern and modulates the transmission signal, as described above.

The pulser applies the transmitting pulse corresponding to the modulation signal to the transducer array (operation 920).

As illustrated in FIG. 15, the synthesized modulation signal output from the modulator is input to the pulsers, which are equal in number to the elements of the transducer array. The pulser outputs the transmitting pulse corresponding to the synthesized modulation signal and applies the output to the transducer element.

According to the ultrasound probe, ultrasound diagnostic apparatus having the same and method of generating an ultrasound signal of exemplary embodiments, it is possible to increase matching of a transmission signal with respect to a frequency spectrum of a transducer element.

Also, it is possible to increase transmission power efficiency and a signal-to-noise ratio of the transmission signal.

The foregoing exemplary embodiments and advantages are merely exemplary and are not to be construed as limiting. Also, the description of the exemplary embodiments is intended to be illustrative, and not to limit the scope of the claims, and many alternatives, modifications, and variations will be apparent to those skilled in the art.

**Claims**

1. An ultrasound probe comprising:

    a transmission unit configured to generate a transmission signal; and
    a modulator configured to generate a modulation signal having a pulse width proportional to a size of an amplitude of the transmission signal at a peak level and a base level for each period of the transmission signal.

2. The ultrasound probe according to claim 1, wherein the transmission unit is configured to output the transmission signal in which a first transmission signal and a second transmission signal that is shifted by a time delay from the first transmission signal are synthesized.

3. The ultrasound probe according to claim 1, wherein the transmission unit includes:

    a first transmission element configured to output a first transmission signal; and
    a second transmission element configured to output a second transmission signal that is shifted by a predetermined time from the first transmission signal.

4. The ultrasound probe according to claim 2, wherein the second transmission signal is shifted from the first transmission signal by the time delay corresponding to 1/4 of a period of the first transmission signal.

5. The ultrasound probe according to claim 3, wherein the modulator is configured to generate a modulation signal having a pulse width proportional to a size of an amplitude of a synthesized signal at a peak level and a base level for each period of the signal in which the first transmission signal and the second transmission signal are synthesized.

6. The ultrasound probe according to claim 3, wherein the modulator includes:

    a first modulator configured to generate a first modulation signal having a pulse width proportional to a size of an amplitude of the first transmission signal at a peak level and a base level for each period of the first transmission signal; and
    a second modulator configured to generate a second modulation signal having a pulse width proportional to a size of an amplitude of the second transmission signal at a peak level and a base level for each period of the second transmission signal.

7. The ultrasound probe according to claim 6, further comprising:

    an adder configured to synthesize the first modulation signal and the second modulation signal.

8. The ultrasound probe according to claim 1, further comprising:

    a pulser configured to generate a transmitting pulse corresponding to the modulation signal and apply the pulse to a transducer array.

9. A method of generating an ultrasound signal, the method comprising:

generating a transmission signal; and

generating a modulation signal having a pulse width proportional to a size of an amplitude of the transmission signal at a peak level and a base level for each period of the transmission signal.

10. The method according to claim 9, wherein the generating the transmission signal includes:

generating a first transmission signal;

generating a second transmission signal that is shifted by a time delay from the first transmission signal; and

generating the transmission signal by synthesizing the first transmission signal and the second transmission signal.

11. The method according to claim 10, wherein the generating the shifted second transmission signal includes:

generating the second transmission signal that is shifted by the time delay corresponding to 1/4 of a period of the first transmission signal.

12. The method according to claim 9, further comprising:

generating a signal that is shifted by a predetermined time from the transmission signal;

generating a shifted modulation signal having a pulse width proportional to a size of an amplitude of the shifted signal at a peak level and a base level for each period of the shifted signal; and

generating a synthesized modulation signal by synthesizing the modulation signal and the shifted modulation signal.

13. The method according to claim 12, wherein the generating the shifted signal includes:

generating a signal that is shifted by a time corresponding to 1/4 of a period of the transmission signal.

14. The method according to claim 9, further comprising:

generating a transmitting pulse corresponding to the modulation signal and applying the transmitting pulse to a transducer array.

# FIG. 1

**FIG. 2**

**FIG. 3**

**FIG. 4**

# FIG.5

ELEVATION
DIRECTION(y)

LATERAL
DIRECTION(x)

AXIAL
DIRECTION(z)

502

TRANSDUCER
ELEMENT

SCAN
DIRECTION

LAST SCANLINE

M-th SCANLINE

FIRST SCANLINE

FIG. 6

EP 3 048 454 A1

**FIG. 7**

EP 3 048 454 A1

110

$w(t-\tau) \cdot \cos[\theta(t-\tau)+\phi]$

$w(t-\tau_1)\cos[\theta(t-\tau_1)+\phi]$    $\cos[\theta(t-\tau_1)]$

CENTER
FREQUENCY
($f_0$)

516

514

510

508

506

| APODIZATION | SECOND DELAY TIME ($\tau_2$) | ⊗ | PHASE SHIFT ($\phi$) | GENERATE SINUSOIDAL WAVE ($\cos[\theta(t)]$) | FIRST DELAY TIME ($\tau_1$) |

$A \cdot w(t-\tau) \cdot \cos[\theta(t-\tau)+\phi]$

512

504

$w(t-\tau_1)$

| WINDOW FUNCTION {$w(t)$} | START TRANSMISSION BEAM |

# FIG. 8

# FIG.9A

EP 3 048 454 A1

FIG.9B

25

FIG.10

# FIG.11

FIG. 12

EP 3 048 454 A1

**FIG. 13**

# FIG.14

100

TA

10          112          560          111

TRANSMISSION
T/R SWITCH ← PULSER ← MODULATOR ← ELEMENT

M x N = K

**FIG. 15**

$g'(t)$ {$= g(t)+g(t-t_0)$}

100

TRANSMISSION ELEMENT

MODULATOR

$g_m'(t)$

112-1 FIRST PULSER

112-2 SECOND PULSER

112-k K TH PULSER

111

560

TA

$M \times N = K$

EP 3 048 454 A1

# FIG.16

200

TA

10

220

210

```
TA
M x N = K
```

| T/R SWITCH | → | RECEPTION SIGNAL PROCESSOR | → | RECEPTION UNIT |

# FIG. 17

TA

M x N = K

220-1
1ST RECEPTION
SIGNAL
PROCESSOR

220-2
2ND RECEPTION
SIGNAL
PROCESSOR

3RD RECEPTION
SIGNAL
PROCESSOR
220-3

220(k-1)
(k-1)TH
RECEPTION
SIGNAL
PROCESSOR

220-K
K TH
RECEPTION
SIGNAL
PROCESSOR

200

210
RECEPTION
UNIT

1

2

3

K

## FIG. 18

FOCUSED SIGNAL

sum

TIME-DELAYED RECEPTION SIGNAL

TIME DELAY

RECEPTION SIGNAL

TRANSDUCER ELEMENTS

FOCAL POINT

ECHO SIGNAL

# FIG.19

190

P

TA

M x N

170
TRANSMITTER

270
RECEIVER

8

500
CONTROLLER

540
INPUT UNIT

DISPLAY

550

530
IMAGE PROCESSOR

# FIG.20

# FIG.21

# FIG.22

START

GENERATE FIRST TRANSMISSION SIGNAL
AND SECOND TRANSMISSION SIGNAL THAT
IS SHIFTED BY PREDETERMINED TIME FROM
FIRST TRANSMISSION SIGNAL ———700

GENERATE FIRST MODULATION SIGNAL AND
SECOND MODULATION SIGNAL HAVING PULSE
WIDTH PROPORTIONAL TO SIZE OF AMPLITUDE
OF FIRST TRANSMISSION SIGNAL AND
SECOND TRANSMISSION SIGNAL, RESPECTIVELY ———710

SYNTHESIZE FIRST MODULATION SIGNAL
AND SECOND MODULATION SIGNAL ———720

APPLY TRANSMITTING PULSE CORRESPONDING
TO SYNTHESIZED MODULATION SIGNAL
TO TRANSDUCER ARRAY ———730

END

# FIG.23

START

↓

| GENERATE FIRST TRANSMISSION SIGNAL AND SECOND TRANSMISSION SIGNAL THAT IS SHIFTED BY PREDETERMINED TIME FROM FIRST TRANSMISSION SIGNAL | 800 |

↓

| SYNTHESIZE FIRST TRANSMISSION SIGNAL AND SECOND TRANSMISSION SIGNAL | 810 |

↓

| GENERATE MODULATION SIGNAL HAVING PULSE WIDTH PROPORTIONAL TO SIZE OF AMPLITUDE OF SYNTHESIZED TRANSMISSION SIGNAL | 820 |

↓

| APPLY TRANSMITTING PULSE CORRESPONDING TO MODULATION SIGNAL TO TRANSDUCER ARRAY | 830 |

↓

END

# FIG.24

START

GENERATE TRANSMISSION SIGNAL IN WHICH
FIRST SIGNAL AND SECOND SIGNAL THAT
IS SHIFTED BY PREDETERMINED TIME FROM
FIRST SIGNAL ARE SYNTHESIZED ⌇900

GENERATE MODULATION SIGNAL HAVING
PULSE WIDTH PROPORTIONAL TO SIZE OF
AMPLITUDE OF TRANSMISSION SIGNAL ⌇910

APPLY TRANSMITTING PULSE CORRESPONDING
TO MODULATION SIGNAL TO TRANSDUCER ARRAY ⌇920

END

**EUROPEAN SEARCH REPORT**

Europäisches Patentamt
European Patent Office
Office européen des brevets

Application Number

EP 15 18 2839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 833 614 A (DODD STIRLING S [US] ET AL) 10 November 1998 (1998-11-10)<br>* column 2, line 64 - column 3, line 28 *<br>* column 7, line 22 - column 8, line 52 *<br>* figures 1,4-6 *<br>----- | 1-14 | INV.<br>G01S7/52<br>G01S15/10 |
| X | US 2004/254459 A1 (KRISTOFFERSEN KJELL [NO] ET AL) 16 December 2004 (2004-12-16)<br>* paragraphs [0049], [0050] *<br>* figure 9 *<br>----- | 1,9 | |

TECHNICAL FIELDS
SEARCHED (IPC)

G01S

The present search report has been drawn up for all claims

| Place of search | Date of completion of the search | Examiner |
|---|---|---|
| Munich | 6 June 2016 | Willig, Hendrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 18 2839

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2016

| Patent document cited in search report | | Publication date | Patent family member(s) | | Publication date |
|---|---|---|---|---|---|
| US 5833614 | A | 10-11-1998 | AU | 8396898 A | 10-02-1999 |
| | | | DE | 69830589 D1 | 21-07-2005 |
| | | | DE | 69830589 T2 | 04-05-2006 |
| | | | EP | 0999788 A1 | 17-05-2000 |
| | | | JP | 4233749 B2 | 04-03-2009 |
| | | | JP | 2001510065 A | 31-07-2001 |
| | | | US | 5833614 A | 10-11-1998 |
| | | | WO | 9903400 A1 | 28-01-1999 |
| US 2004254459 | A1 | 16-12-2004 | DE | 102004027025 A1 | 30-12-2004 |
| | | | JP | 4942290 B2 | 30-05-2012 |
| | | | JP | 2005000663 A | 06-01-2005 |
| | | | US | 2004254459 A1 | 16-12-2004 |

EPO FORM P0459

For more details about this annex : see Official Journal of the European Patent Office, No. 12/82